# EUROPEAN PATENT SPECIFICATION

(11) **EP 3 611 159 B1**
(45) Date of publication and mention of the grant of the patent: **25.01.2023**
(21) Application number: 19188210.9
(22) Date of filing: 08.07.2014
(51) Int. Cl.: C07C 229/08, C07C 229/22, A61K 31/223, A61P 25/00

(54) **DIHYDROXYPHENYL NEUROTRANSMITTER COMPOUNDS, COMPOSITIONS AND METHODS**
DIHYDROXYPHENYL-NEUROTRANSMITTER-VERBINDUNGEN, ZUSAMMENSETZUNGEN UND VERFAHREN
COMPOSÉS NEUROTRANSMETTEURS DE DIHYDROXYPHÉNYLE, COMPOSITIONS ET PROCÉDÉS

(30) Priority: 08.07.2013 US 201361843549 P; 10.06.2014 US 201462010098 P
(43) Date of publication of application: 19.02.2020
(62) Divisional of application: 14822407.4
(73) Proprietor: Auspex Pharmaceuticals, Inc., Parsippany NJ 07054 (US); The U.S.A. as represented by the Secretary, Department of Health and Human Services, Bethesda, MD 20892-7660 (US)
(72) Inventor: GOLDSTEIN, David, S., Bethesda, MD 20892 (US); HOLMES, Courtney, Bethesda, MD 20892 (US); ALKEN, Rudolf-Giesbert, Parsippany, New Jersey 07054 (US); SCHNEIDER, Frank, Parsippany, New Jersey 07054 (US); ZHANG, Chengzhi, Parsippany, New Jersey 07054 (US)
(74) Representative: D Young & Co LLP

(56) References cited:
- WO-A2-2008/112773
- WO-A2-2010/054286
- GOLDSTEIN DAVID S: "L-dihydroxyphenylserine (L-DOPS): A norepinephrine prodrug", CARDIOVASCULAR DRUG REVIEWS, NEVA PRESS, BRANFORD, CT, US, vol. 24, no. 3-4, 1 October 2006 (2006-10-01), pages 189-203, XP009107442, ISSN: 0897-5957, DOI: 10.1111/J.1527-3466.2006.00189.X

## Description

This application claims the benefit of priority of United States provisional applications No. 62/010,098, filed June 10, 2014, and No. 61/843,549, filed July 8, 2013.

Disclosed herein are new dihydoxyphenyl compounds and compositions and their application as pharmaceuticals for the treatment of disorders. Methods of modulating neurotransmitter levels in a subject are also provided for the treatment of disorders such as hypotension, orthostatic hypotension, neurogenic orthostatic hypotension, symptomatic neurogenic orthostatic hypotension, neurogenic orthostatic hypotension associated with multiple sytem atrophy (MSA), orthostatic hypotension associated with Shy-Drager syndrome, neurogenic orthostatic hypotension associated with familial amyloid polyneuropathy (FAP), neurogenic orthostatic hypotension associated with pure autonomic failure (PAF), idiopathic orthostatic hypotension, asympathicotonic hypotension, neurogenic orthostatic hypotension associated with Parkinson's disease, intradialytic hypotension (IDH), hemodialysis-induced hypotension, hypotension associated with fibromyalgia syndrome (FMS), hypotension in spinal cord injury, hypotension associated with chronic fatigue syndrome (CFS), frozen gait, akinesia, and dysarthria in Parkinson's disease, Lewy body dementia, rapid eye movement (REM) behavior disorder, chronic heart failure, stress-related disorders, motor or speech disturbances, chronic pain, stroke, cerebral ischemia, nasal congestion, mood disorders, sleep disorders, narcolepsy, insomnia, attention deficit disorder (ADD), attention deficit hyperactivity disorder (ADHD), anosmia, hyposmia, mild cognitive impairment (MCI), Down syndrome, Alzheimer's disease, postural reflex abnormality caused by Parkinson's disease, autoimmune autonomic failure, familial dysautonomia, diabetic autonomic neuropathy, amyloidosis in the setting of multiple myeloma, Parkinson's disease, proprandial hypotension, dopamine beta-hydroxylase deficiency, pain, progressive supranuclear palsy, Menkes disease, familial dysautonomia (Riley-Day Syndrome), PD-related dysautonomia (autonomic dysfunction), orthostatic intolerance in adolescents, neurocardiogenic syncope (vasovagal), postural orthostatic tachycardia syndrome (POTS), fibromyalgia, allodynia, hyperalgesia, fatigue, sleep disturbance, depression, chronic orthostatic intolerance, pediatric developmental disorders, genetic diseases involving decreased norepinephrine synthesis or effects, multi-system disorders of regulation, pain, neurodegenerative diseases, cognitive dysfunction, olfactory disorders, neuroendocrine disorders, and autoimmune disorders.

Droxidopa (Northera^{®}; DOPS; L-DOPS; L-threo-DOPS; SM 5688; (2S,3R)-3-(3,4-Dihydroxyphenyl)-2-amino-3-hydroxypropanoic acid; or L-threo-dihydroxyphenylserine) is a neurotransmitter modulator. In the body droxidopa is converted to norepinephrine (synonymous with noradrenaline), by the action of the enzyme L-aromatic-amino-acid decarboxylase. Droxidopa therefore is a norepinephrine precursor.

Norepinephrine is an important chemical in the brain and periphery. In the brain norepinephrine is a classic neurotransmitter, thought to be involved in many neurobehavioral phenomena such as attention, memory, wakefulness, and distress. In the periphery norepinephrine is the main neurotransmitter of the sympathetic nervous system responsible for regulation of the circulation.

When a person stands up, the decrease in venous return to the heart unloads baroreceptors and reflexively increases sympathetic nerve traffic. This augments norepinephrine release from sympathetic nerves in the heart and blood vessel walls. The released norepinephrine binds to adrenoceptors and thereby evokes constriction of blood vessels, which helps to maintain blood pressure during orthostasis. Predictably, orthostatic hypotension, a fall in blood pressure when a person stands up, is a cardinal manifestation of sympathetic noradrenergic failure.

A wide variety of both common and rare medical and psychiatric conditions are known or suspected to involve norepinephrine deficiency, because of noradrenergic denervation, failure to synthesize norepinephrine, or inadequate or inappropriate norepinephrine release or inactivation. However, oral norepinephrine is ineffective for treatment of norepinephrine deficiency, because norepinephrine is efficiently metabolized in the gut. Norepinephrine in the portal venous drainage is also extensively metabolized in the liver. Moreover, because of the blood-brain barrier for catecholamines, very little of norepinephrine in the systemic circulation enters the brain unchanged.

In contrast, oral droxidopa enters the bloodstream, and as a neutral amino acid it can traverse the blood-brain barrier. Therefore, droxidopa could be an effective treatment for conditions associated with norepinephrine deficiency.

Droxidopa is approved for use in symptomatic neurogenic orthostatic hypotension. Birkmayer et al., J. Neural Trans., 1983, 58(3-4), 305-13; Freeman et al., Clin. Neuropharmacol., 1991, 14(4), 296-304; Mathias et al., Clinical Autonomic Research: Official J. Clinical Autonomic Research Society, 2001, 11(4), 235-42; Goldstein, Cardiovascular Drug Rev., 2006, 24(3-4), 189-203; Vichayanrat et al., Future Neurology, 2013, 8(4), 381-397; and Hauser et al., J. Parkinson's Disease, 2014, 4(1), 57-65. Droxidopa is currently under investigation for the treatment of neurogenic orthostatic hypotension associated with multiple sytem atrophy (MSA), orthostatic hypotension associated with Shy-Drager syndrome, neurogenic orthostatic hypotension associated with familial amyloid polyneuropathy (FAP), neurogenic orthostatic hypotension associated with pure autonomic failure (PAF), idiopathic orthostatic hypotension, asympathicotonic hypotension, neurogenic orthostatic hypotension associated with Parkinson's disease, intradialytic hypotension (IDH), hemodialysis-induced hypotension, hypotension associated with fibromyalgia syndrome (FMS), hypotension in spinal cord injury, and hypotension associated with chronic fatigue syndrome (CFS). Suzuki et al., Neurology 1981, 31(10), 1323-6; Iida et al., Nephrology, Dialysis, Transplantation: Official Publication of the European Dialysis and Transplant Association - European Renal Association, 1994, 9(8), 1130-5; Freeman et al., Neurology, 1996, 47(6), 1414-20; Wikstrom et al., Amyloid, 1996, 3(3), 162-166; Carvalho et al., J. Autonomic Nervous Syst., 1997, 62(1/2), 63-71; Terazaki et al., J. Autonomic Nervous Syst., 1998, 68(1-2), 101-8; Freeman et al., Neurology, 1999, 53(9), 2151-7; Goldstein et al., Cardiovascular Drug Review, 2006, 24(3-4), 189-203; and Iida et al., Am. J. Nephrology, 2002, 22(4), 338-46. Droxidopa has also shown promise in the treatment of frozen gait, akinesia, and dysarthria in Parkinson's disease, Lewy body dementia, rapid eye movement (REM) behavior disorder, chronic heart failure, stress-related disorders, motor or speech disturbances, chronic pain, stroke, cerebral ischemia, nasal congestion, mood disorders, sleep disorders, narcolepsy, insomnia, attention deficit disorder (ADD), attention deficit hyperactivity disorder (ADHD), anosmia, hyposmia, mild cognitive impairment (MCI), Down syndrome, Alzheimer's disease, and postural reflex abnormality caused by Parkinson's disease, autoimmune autonomic failure, familial dysautonomia, diabetic autonomic neuropathy, amyloidosis in the setting of multiple myeloma, Parkinson's disease, proprandial hypotension, dopamine beta-hydroxylase deficiency, pain, progressive supranuclear palsy, Menkes disease, familial dysautonomia (Riley-Day Syndrome), PD-related dysautonomia (autonomic dysfunction), orthostatic intolerance in adolescents, neurocardiogenic syncope (vasovagal), postural orthostatic tachycardia syndrome (POTS), fibromyalgia, allodynia, hyperalgesia, fatigue, sleep disturbance, and depression. Ogawa et al., J. Medicine, 1985, 16(5-6), 525-34; Yamamoto et al., Clin. Neuropharmacol., 1985, 8(4), 334-42; CA 2133514 A1; Takagi et al., Eur. Neuropsychopharmacol., 1996, 6(1), 43-7; EP 887078 A1; Miyai et al., Neurorehabilitation and Neural Repair, 2000, 14(2), 141-7; WO 2005084330 A2; WO 2008137923 A2; WO 2010132128 A1; WO 2012158612 A1; Kalinin et al., Neurobiology of Aging, 2012, 33(8), 1651-1663; Goldstein et al., Cardiovascular Drug Review, 2006, 24(3-4), 189-203; US 8383681; and US 8008285.

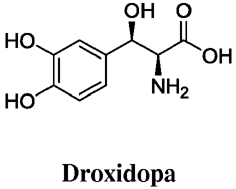

The droxidopa chemical structure contains a number of features that we posit will produce inactive or toxic metabolites, the formation of which can be reduced by the approach described herein. Droxidopa is subject to metabolism by aromatic L-amino acid decarboxylase to give norepinephrine (noradrenaline), which is further methylated by phenylethanolamine N-methyltransferase to give epinephrine (adrenaline). Norepinephrine and epinephrine are subject to oxidative metabolism by monoamine oxidase (MAO) to give the toxic metabolite 3,4-dihydroxyphenylglycolaldehyde (DOPEGAL).

Monoamine oxidase not only limits the potency of droxidopa as a norepinephrine pro-drug but may also lead to toxicity. The immediate product of the action of monoamine oxidase on norepinephrine is the catecholaldehyde, dihydroxyphenylglycolaldehyde. Dihydroxyphenylglycolaldehyde is potentially toxic, by causing cross-linking and thereby inactivation of proteins, as well as auto-oxidation to form harmful quinones. The enzymatic deamination produces hydrogen peroxide, an oxidative stressor.

These, as well as other metabolic transformations, occur in part through polymorphically-expressed enzymes, exacerbating interpatient variability. Additionally, some droxidopa metabolites may have undesirable side effects. Side effects associated with droxidopa administration include headache, dizziness, nausea, hypertension, falls, urinary tract infection, syncope, supine hypertension, hyperpyrexia, confusion, exacerbation of existing ischemic heart disease, arrhythmias, and congestive heart failure. In order to overcome its short half-life, the drug likely must be taken three times daily, which increases the probability of patient incompliance and discontinuance. Further, abruptly stopping treatment with droxidopa can lead to withdrawal or discontinuation syndrome. Medicines with longer half-lives will likely attenuate these deleterious effects.

### Deuterium Kinetic Isotope Effect

In order to eliminate foreign substances such as therapeutic agents, the animal body expresses various enzymes, such as the cytochrome P₄₅₀ enzymes (CYPs), esterases, proteases, reductases, dehydrogenases, and monoamine oxidases, to react with and convert these foreign substances to more polar intermediates or metabolites for renal excretion. Such metabolic reactions frequently involve the oxidation of a carbon-hydrogen (C-H) bond to either a carbon-oxygen (C-O) or a carbon-carbon (C-C) π-bond. The resultant metabolites may be stable or unstable under physiological conditions, and can have substantially different pharmacokinetic, pharmacodynamic, and acute and long-term toxicity profiles relative to the parent compounds. For most drugs, such oxidations are generally rapid and ultimately lead to administration of multiple or high daily doses.

The relationship between the activation energy and the rate of reaction may be quantified by the Arrhenius equation, k = Ae^{-Eact/RT}. The Arrhenius equation states that, at a given temperature, the rate of a chemical reaction depends exponentially on the activation energy (E_{act}).

The transition state in a reaction is a short lived state along the reaction pathway during which the original bonds have stretched to their limit. By definition, the activation energy E_{act} for a reaction is the energy required to reach the transition state of that reaction. Once the transition state is reached, the molecules can either revert to the original reactants, or form new bonds giving rise to reaction products. A catalyst facilitates a reaction process by lowering the activation energy leading to a transition state. Enzymes are examples of biological catalysts.

Carbon-hydrogen bond strength is directly proportional to the absolute value of the ground-state vibrational energy of the bond. This vibrational energy depends on the mass of the atoms that form the bond, and increases as the mass of one or both of the atoms making the bond increases. Since deuterium (D) has twice the mass of protium (¹H), a C-D bond is stronger than the corresponding C-¹H bond. If a C-¹H bond is broken during a rate-determining step in a chemical reaction (i.e. the step with the highest transition state energy), then substituting a deuterium for that protium will cause a decrease in the reaction rate. This phenomenon is known as the Deuterium Kinetic Isotope Effect (DKIE). The magnitude of the DKIE can be expressed as the ratio between the rates of a given reaction in which a C-¹H bond is broken, and the same reaction where deuterium is substituted for protium. The DKIE can range from about 1 (no isotope effect) to very large numbers, such as 50 or more. Substitution of tritium for hydrogen results in yet a stronger bond than deuterium and gives numerically larger isotope effects

Deuterium (²H or D) is a stable and non-radioactive isotope of hydrogen which has approximately twice the mass of protium (¹H), the most common isotope of hydrogen. Deuterium oxide (D₂O or "heavy water") looks and tastes like H₂O, but has different physical properties.

When pure D₂O is given to rodents, it is readily absorbed. The quantity of deuterium required to induce toxicity is extremely high. When about 0-15% of the body water has been replaced by D₂O, animals are healthy but are unable to gain weight as fast as the control (untreated) group. When about 15-20% of the body water has been replaced with D₂O, the animals become excitable. When about 20-25% of the body water has been replaced with D₂O, the animals become so excitable that they go into frequent convulsions when stimulated. Skin lesions, ulcers on the paws and muzzles, and necrosis of the tails appear. The animals also become very aggressive. When about 30% of the body water has been replaced with D₂O, the animals refuse to eat and become comatose. Their body weight drops sharply and their metabolic rates drop far below normal, with death occurring at about 30 to about 35% replacement with D₂O. The effects are reversible unless more than thirty percent of the previous body weight has been lost due to D₂O. Studies have also shown that the use of D₂O can delay the growth of cancer cells and enhance the cytotoxicity of certain antineoplastic agents.

Deuteration of pharmaceuticals to improve pharmacokinetics (PK), pharmacodynamics (PD), and toxicity profiles has been demonstrated previously with some classes of drugs. For example, the DKIE was used to decrease the hepatotoxicity of halothane, presumably by limiting the production of reactive species such as trifluoroacetyl chloride. However, this method may not be applicable to all drug classes. For example, deuterium incorporation can lead to metabolic switching. Metabolic switching occurs when xenogens, sequestered by Phase I enzymes, bind transiently and re-bind in a variety of conformations prior to the chemical reaction (e.g., oxidation). Metabolic switching is enabled by the relatively vast size of binding pockets in many Phase I enzymes and the promiscuous nature of many metabolic reactions. Metabolic switching can lead to different proportions of known metabolites as well as altogether new metabolites. This new metabolic profile may impart more or less toxicity. Such pitfalls are non-obvious and are not predictable *a priori* for any drug class.

Droxidopa is a neurotransmitter modulator. The carbon-hydrogen bonds of droxidopa contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increased levels of deuterium incorporation may produce a detectable Deuterium Kinetic Isotope Effect (DKIE) that could effect the pharmacokinetic, pharmacologic and/or toxicologic profiles of such droxidopa in comparison with the compound having naturally occurring levels of deuterium.

Based on discoveries made in our laboratory, as well as considering the literature, droxidopa is likely metabolized in humans to give epinephrine and norepinephrine, which are further metabolized at their N-methylene group. The current approach has the potential to prevent metabolism at this site. Other sites on the molecule may also undergo transformations leading to metabolites with as-yet-unknown pharmacology/toxicology. Limiting the production of these metabolites has the potential to decrease the danger of the administration of such drugs and may even allow increased dosage and/or increased efficacy. All of these transformations can occur through polymorphically-expressed enzymes, exacerbating interpatient variability. Further, some disorders are best treated when the subject is medicated around the clock or for an extended period of time. For all of the foregoing reasons, a medicine with a longer half-life may result in greater efficacy and cost savings. Various deuteration patterns can be used to (a) reduce or eliminate unwanted metabolites, (b) increase the half-life of the parent drug, (c) decrease the number of doses needed to achieve a desired effect, (d) decrease the amount of a dose needed to achieve a desired effect, (e) increase the formation of active metabolites, if any are formed, (f) decrease the production of deleterious metabolites in specific tissues, and/or (g) create a more effective drug and/or a safer drug for polypharmacy, whether the polypharmacy be intentional or not. The deuteration approach has the strong potential to slow the metabolism of droxidopa and attenuate interpatient variability.

Novel compounds and pharmaceutical compositions, certain of which have been found to function as neurotransmitter prodrugs have been discovered, together with methods of using the compounds, including methods for the treatment of neurotransmitter-mediated disorders in a patient by administering the compounds.

Certain compounds disclosed herein may possess useful neurotransmitter modulating activity, and may be used in the treatment or prophylaxis of a disorder in which neurotransmitter levels play an active role. Thus, certain embodiments also provide pharmaceutical compositions comprising one or more compounds disclosed herein together with a pharmaceutically acceptable carrier, as well as methods of using the compounds and compositions. Certain embodiments provide the compounds for use in methods for modulating neurotransmitter activity. Other embodiments provide the compounds for use in methods for treating a neurotransmitter -mediated disorder in a patient in need of such treatment, comprising administering to said patient a therapeutically effective amount of a compound or composition according to the present invention. Also provided is the use of certain compounds disclosed herein for use in the manufacture of a medicament for the prevention or treatment of a disorder ameliorated by the modulation of neurotransmitter levels.

The compounds as disclosed herein may also contain less prevalent isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen.

In certain embodiments, the compound disclosed herein may expose a patient to a maximum of about 0.000005% D₂O or about 0.00001% DHO, assuming that all of the C-D bonds in the compound as disclosed herein are metabolized and released as D₂O or DHO. In certain embodiments, the levels of D₂O shown to cause toxicity in animals is much greater than even the maximum limit of exposure caused by administration of the deuterium enriched compound as disclosed herein. Thus, in certain embodiments, the deuterium-enriched compound disclosed herein should not cause any additional toxicity due to the formation of D₂O or DHO upon drug metabolism.

In certain embodiments, said compound is not enriched by carbon-13. The invention is defined in claim 1.

In certain embodiments, said compound has the structural formula:

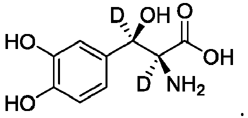

In certain embodiments, the deuterated compounds disclosed herein maintain the beneficial aspects of the corresponding non-isotopically enriched molecules while substantially increasing the maximum tolerated dose, decreasing toxicity, increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions.

In certain embodiments, disclosed herein is an extended-release pharmaceutical formulation comprising, in a solid dosage form for oral delivery of between about 100 mg and about 1 g total weight:
between about 2 and about 18% of a compound as disclosed herein;
between about 70% and about 96% of one or more diluents;
between about 1% and about 10% of a water-soluble binder ; and
between about 0.5 and about 2% of a surfactant.

In certain embodiments, the diluent or diluents are chosen from mannitol, lactose, and microcrystalline cellulose; the binder is a polyvinylpyrrolidone; and the surfactant is a polysorbate.

In certain embodiments, the extended-release pharmaceutical formulation comprises between about 2.5% and about 11% of a compound as disclosed herein.

In certain embodiments, the extended-release pharmaceutical formulation comprises:
between about 60% and about 70% mannitol or lactose;
between about 15% and about 25% microcrystalline cellulose
about 5% of polyvinylpyrrolidone K29/32; and
between about 1 and about 2% of Tween 80.

In certain embodiments, the extended-release pharmaceutical formulation comprises:
between about 4% and about 9% of a compound as disclosed herein;
between about 60% and about 70% mannitol or lactose;
between about 20% and about 25% microcrystalline cellulose
about 5% of polyvinylpyrrolidone K29/32; and
about 1.4% of Tween 80.

In certain embodiments, disclosed herein is an extended-release pharmaceutical formulation comprising, in a solid dosage form for oral delivery of between about 100 mg and about 1 g total weight:
between about 70 and about 95% of a granulation of a compound as disclosed herein, wherein the active ingredient comprises between about 1 and about 15% of the granulation;
between about 5% and about 15% of one or more diluents;
between about 5% and about 20% of sustained-release polymer; and
between about 0.5 and about 2% of a lubricant.

In certain embodiments, the extended-release pharmaceutical formulation comprises:
between about 5% and about 15% of one or more spray-dried mannitol or spray-dried lactose;
between about 5% and about 20% of sustained-release polymer; and
between about 0.5 and about 2% of a magnesium stearate.

In certain embodiments, the sustained-release polymer is chosen from a polyvinyl acetate-polyvinylpyrrolidone mixture and a poly(ethylene oxide) polymer.

In certain embodiments, the sustained-release polymer is chosen from Kollidon^{®} SR, POLYOX^{®} N60K, and Carbopol^{®}.

In certain embodiments, the sustained-release polymer is Kollidon^{®} SR.

In certain embodiments, the sustained-release polymer is POLYOX^{®} N60K.

In certain embodiments, the sustained-release polymer is Carbopol^{®}.

In certain embodiments, the extended-release pharmaceutical formulation comprises from about 5 mg to about 100 mg of a compound as disclosed herein.

In certain embodiments, the compounds disclosed herein can be formulated as extended-release pharmaceutical formulations as described in U.S. Patent Application No. 14/030,322, filed September 18, 2013.

As used herein, the terms below have the meanings indicated.

The singular forms "a," "an," and "the" may refer to plural articles unless specifically stated otherwise.

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

When ranges of values are disclosed, and the notation "from n₁ ... to n₂" or "n₁-n₂" is used, where n₁ and n₂ are the numbers, then unless otherwise specified, this notation is intended to include the numbers themselves and the range between them. This range may be integral or continuous between and including the end values.

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be determined using conventional analytical methods known to one of ordinary skill in the art, including mass spectrometry and nuclear magnetic resonance spectroscopy.

The term "is/are deuterium," when used to describe a given position in a molecule such as R₁-R₁₁ or the symbol "D", when used to represent a given position in a drawing of a molecular structure, means that the specified position is enriched with deuterium above the naturally occurring distribution of deuterium. In one embodiment deuterium enrichment is no less than about 1%, in another no less than about 5%, in another no less than about 10%, in another no less than about 20%, in another no less than about 50%, in another no less than about 70%, in another no less than about 80%, in another no less than about 90%, or in another no less than about 98% of deuterium at the specified position.

The term "isotopic enrichment" refers to the percentage of incorporation of a less prevalent isotope of an element at a given position in a molecule in the place of the more prevalent isotope of the element.

The term "non-isotopically enriched" refers to a molecule in which the percentages of the various isotopes are substantially the same as the naturally occurring percentages.

Asymmetric centers exist in the compounds disclosed herein. These centers are designated by the symbols "R" or "S," depending on the configuration of substituents around the chiral carbon atom. It should be understood that the invention encompasses all stereochemical isomeric forms, including diastereomeric, enantiomeric, and epimeric forms, as well as d-isomers and 1-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns, or any other appropriate method known in the art. Starting compounds of particular stereochemistry are either commercially available or can be made and resolved by techniques known in the art. Additionally, the compounds disclosed herein may exist as geometric isomers. The present invention includes all cis, trans, syn, anti, entgegen (E), and zusammen (Z) isomers as well as the appropriate mixtures thereof. Additionally, compounds may exist as tautomers; all tautomeric isomers are provided by this invention. Additionally, the compounds disclosed herein can exist in unsolvated as well as solvated forms with pharmaceutically acceptable solvents such as water, ethanol, and the like. In general, the solvated forms are considered equivalent to the unsolvated forms.

The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position.

The term "disorder" as used herein is intended to be generally synonymous, and is used interchangeably with, the terms "disease" and "condition" (as in medical condition), in that all reflect an abnormal condition of the human or animal body or of one of its parts that impairs normal functioning, is typically manifested by distinguishing signs and symptoms.

The terms "treat," "treating," and "treatment" are meant to include alleviating or abrogating a disorder or one or more of the symptoms associated with a disorder; or alleviating or eradicating the cause(s) of the disorder itself. As used herein, reference to "treatment"of a disorder is intended to include prevention. The terms "prevent," "preventing," and "prevention" refer to a method of delaying or precluding the onset of a disorder; and/or its attendant symptoms, barring a subject from acquiring a disorder or reducing a subject's risk of acquiring a disorder.

The term "therapeutically effective amount" refers to the amount of a compound that, when administered, is sufficient to prevent development of, or alleviate to some extent, one or more of the symptoms of the disorder being treated. The term "therapeutically effective amount" also refers to the amount of a compound that is sufficient to elicit the biological or medical response of a cell, tissue, system, animal, or human that is being sought by a researcher, veterinarian, medical doctor, or clinician.

The term "subject" refers to an animal, including, but not limited to, a primate (e.g., human, monkey, chimpanzee, gorilla, and the like), rodents (e.g., rats, mice, gerbils, hamsters, ferrets, and the like), lagomorphs, swine (e.g., pig, miniature pig), equine, canine, feline, and the like. The terms "subject" and "patient" are used interchangeably herein in reference, for example, to a mammalian subject, such as a human patient.

The term "combination therapy" means the administration of two or more therapeutic agents to treat a therapeutic disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the disorders described herein.

The term "neurotransmitter" refers to endogenous chemicals that transmit signals across a synapse from one neuron (brain cell) to another 'target' neuron. Neurotransmitters are packaged into synaptic vesicles clustered beneath the membrane in the axon terminal, on the presynaptic side of a synapse. Neurotransmitters are released into and diffuse across the synaptic cleft, where they bind to specific receptors in the membrane on the postsynaptic side of the synapse. Many neurotransmitters are synthesized from plentiful and simple precursors, such as amino acids, which are readily available from the diet and which require only a small number of biosynthetic steps to convert. Specific neurotransmitters whose levels are modulated by the compounds disclosed herein include norepinephrine and epinephrine.

Norepinephrine is a catecholamine with multiple roles including those as a hormone and a neurotransmitter. Medically it is used in those with severe hypotension. It does this by increasing vascular tone (tension of vascular smooth muscle) through α-adrenergic receptor activation. One of the most important functions of norepinephrine is its role as the neurotransmitter released from the sympathetic neurons to affect the heart. An increase in norepinephrine from the sympathetic nervous system increases the rate of contractions in the heart. As a stress hormone, norepinephrine affects parts of the brain, such as the amygdala, where attention and responses are controlled. Norepinephrine also underlies the fight-or-flight response, along with epinephrine, directly increasing heart rate, triggering the release of glucose from energy stores, and increasing blood flow to skeletal muscle. It increases the brain's oxygen supply. Norepinephrine is synthesized from dopamine by dopamine β-hydroxylase in the secretory granules of the medullary chromaffin cells. It is released from the adrenal medulla into the blood as a hormone, and is also a neurotransmitter in the central nervous system and sympathetic nervous system, where it is released from noradrenergic neurons in the locus coeruleus. The actions of norepinephrine are carried out via the binding to adrenergic receptors.

Epinephrine is a is a hormone and a neurotransmitter which acts on nearly all body tissues. Its actions vary by tissue type and tissue expression of adrenergic receptors. For example, high levels of epinephrine causes smooth muscle relaxation in the airways but causes contraction of the smooth muscle that lines most arterioles. Epinephrine acts by binding to a variety of adrenergic receptors. Epinephrine is a nonselective agonist of all adrenergic receptors, including the major subtypes α1, α2, β1, β2, and β3. Epinephrine's binding to these receptors triggers a number of metabolic changes. Binding to α-adrenergic receptors inhibits insulin secretion by the pancreas, stimulates glycogenolysis in the liver and muscle, and stimulates glycolysis in muscle. β-Adrenergic receptor binding triggers glucagon secretion in the pancreas, increased adrenocorticotropic hormone (ACTH) secretion by the pituitary gland, and increased lipolysis by adipose tissue. Together, these effects lead to increased blood glucose and fatty acids, providing substrates for energy production within cells throughout the body. Adrenaline is used to treat a number of conditions including: cardiac arrest, anaphylaxis, and superficial bleeding.

The term "neurotransmitter-mediated disorder," refers to a disorder that is characterized by abnormal or suboptimal levels of norepinephrine and/or epinephrine. A neurotransmitter-mediated disorder may be completely or partially mediated by modulating neurotransmitter levels. In particular, a neurotransmitter-mediated disorder is one in which modulation of neurotransmitter levels results in some effect on the underlying disorder e.g., administration of a neurotransmitter modulator results in some improvement in at least some of the patients being treated. In some embodiments the term "neurotransmitter-mediated disorder" refers to a disorder in which there is decreased synthesis, storage, release, reuptake, metabolism, or effect of norepinephrine, such as Parkinson's disease and idiopathic orthostatic hypotension. In some embodiments the term "neurotransmitter-mediated disorder" refers to a disorder that involves low blood pressure, inadequate vasoconstriction, low blood volume, or other situations in which norepinephrine is approved as a drug. In some embodiments the term "neurotransmitter-mediated disorder" refers to a disorder

The term "neurotransmitter level modulator," refers to the ability of a compound disclosed herein to alter levels of norepinephrine and/or epinephrine. An modulator may increase neurotransmitter levels by acting as a biosynthetic precursor to norepinephrine and/or epinephrine. Such modulation may be manifest only in particular cell types or may be contingent on a particular biological event. In some embodiments, modulation of neurotransmitter levels may be assessed using the methods described in Verhagen-Kamerbeek et al., Monit. Mol. Neurosci., Proc. Int. Conf. In Vivo Methods, 5th, 1991, 373-6; Yue et al., J. Pharmacy and Pharmacol., 1992, 44(12), 990-5; and Coll Mar et al., Hepatology (Baltimore, Md.), 2012, 56(5), 1849-60.

The term "therapeutically acceptable" refers to those compounds (or salts, prodrugs, tautomers, zwitterionic forms, etc.) which are suitable for use in contact with the tissues of patients without excessive toxicity, irritation, allergic response, immunogenecity, are commensurate with a reasonable benefit/risk ratio, and are effective for their intended use.

The term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenecity, or other problems or complications, commensurate with a reasonable benefit/risk ratio. *See,* Remington: The Science and Practice of Pharmacy, 21st Edition; Lippincott Williams & Wilkins: Philadelphia, PA, 2005; Handbook of Pharmaceutical Excipients, 5th Edition; Rowe et al., Eds., The Pharmaceutical Press and the American Pharmaceutical Association: 2005; and Handbook of Pharmaceutical Additives, 3rd Edition; Ash and Ash Eds., Gower Publishing Company: 2007; Pharmaceutical Preformulation and Formulation, Gibson Ed., CRC Press LLC: Boca Raton, FL, 2004).

The terms "active ingredient," "active compound," and "active substance" refer to a compound, which is administered, alone or in combination with one or more pharmaceutically acceptable excipients or carriers, to a subject for treating, preventing, or ameliorating one or more symptoms of a disorder.

The terms "drug," "therapeutic agent," and "chemotherapeutic agent" refer to a compound, or a pharmaceutical composition thereof, which is administered to a subject for treating, preventing, or ameliorating one or more symptoms of a disorder.

The term "release controlling excipient" refers to an excipient whose primary function is to modify the duration or place of release of the active substance from a dosage form as compared with a conventional immediate release dosage form.

The term "nonrelease controlling excipient" refers to an excipient whose primary function do not include modifying the duration or place of release of the active substance from a dosage form as compared with a conventional immediate release dosage form.

The term "groups that are easily hydrolytically or enzymatically cleavable under physiological conditions " refers to common protective groups which are used in synthesis or that are such protective groups which lead to so-called prodrugs and are known to those skilled in the art. These groups may be selected from the group comprising methyl, perdeuteromethyl, ethyl, perdeuteroethyl, propyl, perdeuteropropyl, butyl, perdeuterobutyl, C₁ to C₆-alkyl, that may be branched or unbranched, or C₅ to C₆-cycloalkyl, deuterated or partly deuterated C₁ to C₆-alkyl, that may be branched or unbranched, or deuterated or partly deuterated C₅ to C₆-cycloalkyl.

The term "prodrug" refers to a compound functional derivative of the compound as disclosed herein and is readily convertible into the parent compound in vivo. Prodrugs are often useful because, in some situations, they may be easier to administer than the parent compound. They may, for instance, be bioavailable by oral administration whereas the parent compound is not. The prodrug may also have enhanced solubility in pharmaceutical compositions over the parent compound. A prodrug may be converted into the parent drug by various mechanisms, including enzymatic processes and metabolic hydrolysis. See Harper, Progress in Drug Research 1962, 4, 221-294; Morozowich et al. in "Design of Biopharmaceutical Properties through Prodrugs and Analogs," Roche Ed., APHA Acad. Pharm. Sci. 1977; "Bioreversible Carriers in Drug in Drug Design, Theory and Application," Roche Ed., APHA Acad. Pharm. Sci. 1987; "Design of Prodrugs," Bundgaard, Elsevier, 1985; Wang et al., Curr. Pharm. Design 1999, 5, 265-287; Pauletti et al., Adv. Drug. Delivery Rev. 1997, 27, 235-256; Mizen et al., Pharm. Biotech. 1998, 11, 345-365; Gaignault et al., Pract. Med. Chem. 1996, 671-696; Asgharnejad in "Transport Processes in Pharmaceutical Systems," Amidon et al., Ed., Marcell Dekker, 185-218, 2000; Balant et al., Eur. J. Drug Metab. Pharmacokinet. 1990, 15, 143-53; Balimane and Sinko, Adv. Drug Delivery Rev. 1999, 39, 183-209; Browne, Clin. Neuropharmacol. 1997, 20, 1-12; Bundgaard, Arch. Pharm. Chem. 1979, 86, 1-39; Bundgaard, Controlled Drug Delivery 1987, 17, 179-96; Bundgaard, Adv. Drug Delivery Rev.1992, 8, 1-38; Fleisher et al., Adv. Drug Delivery Rev. 1996, 19, 115-130; Fleisher et al., Methods Enzymol. 1985, 112, 360-381; Farquhar et al., J. Pharm. Sci. 1983, 72, 324-325; Freeman et al., J. Chem. Soc., Chem. Commun. 1991, 875-877; Friis and Bundgaard, Eur. J. Pharm. Sci. 1996, 4, 49-59; Gangwar et al., Des. Biopharm. Prop. Prodrugs Analogs, 1977, 409-421; Nathwani and Wood, Drugs 1993, 45, 866-94; Sinhababu and Thakker, Adv. Drug Delivery Rev. 1996, 19, 241-273; Stella et al., Drugs 1985, 29, 455-73; Tan et al., Adv. Drug Delivery Rev. 1999, 39, 117-151; Taylor, Adv. Drug Delivery Rev. 1996, 19, 131-148; Valentino and Borchardt, Drug Discovery Today 1997, 2, 148-155; Wiebe and Knaus, Adv. Drug Delivery Rev. 1999, 39, 63-80; Waller et al., Br. J. Clin. Pharmac. 1989, 28, 497-507.

The compounds disclosed herein can exist as therapeutically acceptable salts. The term "therapeutically acceptable salt," as used herein, represents salts or zwitterionic forms of the compounds disclosed herein which are therapeutically acceptable as defined herein. The salts can be prepared during the final isolation and purification of the compounds or separately by reacting the appropriate compound with a suitable acid or base. Therapeutically acceptable salts include acid and basic addition salts. For a more complete discussion of the preparation and selection of salts, refer to "Handbook of Pharmaceutical Salts, Properties, and Use," Stah and Wermuth, Ed.;( Wiley-VCH and VHCA, Zurich, 2002) and Berge et al., J. Pharm. Sci. 1977, 66, 1-19.

Suitable acids for use in the preparation of pharmaceutically acceptable salts include, but are not limited to, acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, boric acid, (+)-camphoric acid, camphorsulfonic acid, (+)-(1S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, undecylenic acid, and valeric acid.

For the production of the physiologically acceptable salts of the compounds disclosed herein, the usual physiologically acceptable inorganic and organic acids such as hydrochloric acid, hydrobromic acid, phosphoric acid, sulfuric acid, oxalic acid, maleic acid, fumaric acid, lactic acid, tartaric acid, malic acid, citric acid, salicylic acid, adipic acid and benzoic acid can be used, as well as salts with suitable zwitterions (like lysinate and aspartate). Additional acids that can be used are described, for example, in Fortschritte der Arzneimittelforschung, Vol. 10, pp. 224-225, Birkhäuser Publishers, Basel and Stuttgart, 1966, and Journal of Pharmaceutical Sciences, Vol. 66, pp. 1-5 (1977).

The acid addition salts are usually obtained in a way known in and of itself by mixing the free base or solutions thereof with the corresponding acid or solutions thereof in an organic solvent, for example, a lower alcohol, such as methanol, ethanol, n-propanol or isopropanol or a lower ketone such as acetone, methyl ethyl ketone or methyl isobutyl ketone or an ether such as diethyl ether, tetrahydrofuran or dioxane. For better crystal precipitation, mixtures of the named solvents can also be used. In addition, physiologically acceptable aqueous solutions of acid addition salts of the compounds used according to the invention can be produced there from in an aqueous acid solution.

The acid addition salts of the compounds disclosed herein can be converted to the free base in a way known in and of itself, e.g., with alkalis or ion exchangers. Additional salts can be obtained from the free base by reaction with inorganic or organic acids, particularly those which are suitable for the formation of salts that can be employed therapeutically. These or also other salts of the new compound, such as, e.g., the picrate, may also serve for purification of the free base by converting the free base into a salt, separating this salt, and again releasing the base from the salt.

Suitable bases for use in the preparation of pharmaceutically acceptable salts, including, but not limited to, inorganic bases, such as magnesium hydroxide, calcium hydroxide, potassium hydroxide, zinc hydroxide, or sodium hydroxide; and organic bases, such as primary, secondary, tertiary, and quaternary, aliphatic and aromatic amines, including L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methyl-glucamine, hydrabamine, 1H-imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, secondary amines, triethanolamine, trimethylamine, triethylamine, N-methyl-D-glucamine, 2-amino-2-(hydroxymethyl)-1,3-propanediol, and tromethamine.

While it may be possible for the compounds of the subject invention to be administered as the raw chemical, it is also possible to present them as a pharmaceutical composition. Accordingly, provided herein are pharmaceutical compositions which comprise one or more of certain compounds disclosed herein, or one or more pharmaceutically acceptable salts, prodrugs, or solvates thereof, together with one or more pharmaceutically acceptable carriers thereof and optionally one or more other therapeutic ingredients. Proper formulation is dependent upon the route of administration chosen. Any of the well-known techniques, carriers, and excipients may be used as suitable and as understood in the art; *e.g.,* in Remington's Pharmaceutical Sciences. The pharmaceutical compositions disclosed herein may be manufactured in any manner known in the art, *e.g.,* by means of conventional mixing, dissolving, granulating, dragee-making, levigating, emulsifying, encapsulating, entrapping or compression processes. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see, Remington: The Science and Practice of Pharmacy,* supra; Modified-Release Drug Deliver Technology, Rathbone et al., Eds., Drugs and the Pharmaceutical Science, Marcel Dekker, Inc.: New York, NY, 2002; Vol. 126; Hager's Handbuch [Handbook] (5th ed.) 2, 622-1045; List et al., Arzneiformenlehre [Instructions for Drug Forms], Stuttgart: Wiss. Verlagsges. 1985; Sucker et al., Pharmazeutische Technologie [Pharmaceutical Technology], Stuttgart: Thieme 1991; Ullmann's Enzyklopädie [Encyclopedia] (5th ed.) A 19, 241-271; Voigt, Pharmazeutische Technologie [Pharmaceutical Technology], Berlin: Ullstein Mosby 1995).

The compositions include those suitable for oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The compositions may conveniently be presented in unit dosage form and may be prepared by any of the methods well known in the art of pharmacy. Typically, these methods include the step of bringing into association a compound of the subject invention or a pharmaceutically salt, or solvate thereof ("active ingredient") with the carrier which constitutes one or more accessory ingredients. In general, the compositions are prepared by uniformly and intimately bringing into association the active ingredient with liquid carriers or finely divided solid carriers or both and then, if necessary, shaping the product into the desired formulation.

The compositions include those suitable for oral administration. The compositions may conveniently be presented in unit dosage form and may be prepared by any of the methods well known in the art of pharmacy. Typically, these methods include the step of bringing into association a compound of the subject invention or a pharmaceutically salt, prodrug, or solvate thereof ("active ingredient") with the carrier which constitutes one or more accessory ingredients. In general, the compositions are prepared by uniformly and intimately bringing into association the active ingredient with liquid carriers or finely divided solid carriers or both and then, if necessary, shaping the product into the desired formulation.

Formulations of the compounds disclosed herein suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Solutions or suspensions containing the active substance used according to the invention may additionally contain agents that improve taste, such as saccharin, cyclamate or sugar, as well as, e.g., taste enhancers such as vanilla or orange extract. They may also contain suspension adjuvants such as sodium carboxymethylcellulose or preservatives such as p-hydroxybenzoate. Capsules containing active substances can be produced, for example, by mixing the active substance with an inert vehicle such as lactose or sorbitol and encapsulating this mixture in gelatin capsules. Suitable suppositories can be produced, for example, by mixing with vehicle agents provided therefore, such as neutral fats or polyethylene glycol or derivatives thereof.

In certain embodiments, diluents are selected from the group consisting of mannitol powder, spray dried mannitol, microcrystalline cellulose, lactose, dicalcium phosphate, tricalcium phosphate, starch, pregelatinized starch, compressible sugars, silicified microcrystalline cellulose, and calcium carbonate.

In certain embodiments, surfactants are selected from the group consisting of Tween 80, sodium lauryl sulfate, and docusate sodium.

In certain embodiments, binders are selected from the group consisting of povidone (PVP) K29/32, hydroxypropylcellulose (HPC), hydroxypropylmethylcellulose (HPMC), ethylcellulose (EC), corn starch, pregelatinized starch, gelatin, and sugar.

In certain embodiments, lubricants are selected from the group consisting of magnesium stearate, stearic acid, sodium stearyl fumarate, calcium stearate, hydrogenated vegetable oil, mineral oil, polyethylene glycol, polyethylene glycol 4000-6000, talc, and glyceryl behenate.

In certain embodiments, sustained release polymers are selected from the group consisting of POLYOX^{®} (poly (ethylene oxide), POLYOX^{®} N60K grade, Kollidon^{®} SR, HPMC, HPMC (high viscosity), HPC, HPC (high viscosity), and Carbopol^{®}.

In certain embodiments, extended/controlled release coating are selected from a group of ethylcellulose polymers, such as ETHOCEL^{™} and Surelease^{®} Aqueous Ethylcellulose Dispersions.

In certain embodiments, antioxidants are selected from a group consisting of butylated hydroxyanisole (BHA), butylated hydroxytoluene (BHT), sodium ascorbate, and α-tocopherol.

In certain embodiments, tablet coatings are selected from the group of Opadry^{®} 200, Opadry^{®} II, Opadry^{®} fx, Opadry^{®} amb, Opaglos^{®} 2, Opadry^{®} tm, Opadry^{®}, Opadry^{®} NS, Opalux^{®}, Opatint^{®}, Opaspray^{®}, Nutraficient^{®}.

Preferred unit dosage formulations are those containing an effective dose, as herein below recited, or an appropriate fraction thereof, of the active ingredient.

Compounds may be administered orally at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

The compounds may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

The compounds may also be formulated in rectal compositions such as suppositories or retention enemas, *e.g.,* containing conventional suppository bases such as cocoa butter, polyethylene glycol, or other glycerides.

Certain compounds disclosed herein may be administered topically, that is by non-systemic administration. This includes the application of a compound disclosed herein externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semiliquid preparations suitable for penetration through the skin to the site of inflammation such as gels, liniments, lotions, creams, ointments or pastes, and drops suitable for administration to the eye, ear or nose.

For administration by inhalation, compounds may be delivered from an insufflator, nebulizer pressurized packs or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds according to the invention may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

Preferred unit dosage formulations are those containing an effective dose, as herein below recited, or an appropriate fraction thereof, of the active ingredient.

Compounds may be administered orally or via injection at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

In order to obtain the desired effect, the dose of active principle can vary between 100 and 1500 mg per day in divided doses.

Each single dose can contain from 50 to 1000 mg of active principle, in combination with a pharmaceutical vehicle. This single dose can be administered 1 to 4 times daily.

The amount of active ingredient that may be combined with the carrier materials to produce a single dosage form will vary depending upon the host treated and the particular mode of administration.

The compounds can be administered in various modes, *e.g.* orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the disorder being treated. Also, the route of administration may vary depending on the disorder and its severity.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disorder.

In the case wherein the patient's status does improve, upon the doctor's discretion the administration of the compounds may be given continuously or temporarily suspended for a certain length of time (i.e., a "drug holiday").

Once improvement of the patient's conditions has occurred, a maintenance dose is administered if necessary. Subsequently, the dosage or the frequency of administration, or both, can be reduced, as a function of the symptoms, to a level at which the improved disorder is retained. Patients can, however, require intermittent treatment on a long-term basis upon any recurrence of symptoms.

Neurotransmitter-mediated disorders, include, but are not limited to, hypotension, orthostatic hypotension, neurogenic orthostatic hypotension, symptomatic neurogenic orthostatic hypotension, neurogenic orthostatic hypotension associated with multiple sytem atrophy (MSA), orthostatic hypotension associated with Shy-Drager syndrome, neurogenic orthostatic hypotension associated with familial amyloid polyneuropathy (FAP), neurogenic orthostatic hypotension associated with pure autonomic failure (PAF), idiopathic orthostatic hypotension, asympathicotonic hypotension, neurogenic orthostatic hypotension associated with Parkinson's disease, intradialytic hypotension (IDH), hemodialysis-induced hypotension, hypotension associated with fibromyalgia syndrome (FMS), hypotension in spinal cord injury, hypotension associated with chronic fatigue syndrome (CFS), frozen gait, akinesia, and dysarthria in Parkinson's disease, Lewy body dementia, rapid eye movement (REM) behavior disorder, chronic heart failure, stress-related disorders, motor or speech disturbances, chronic pain, stroke, cerebral ischemia, nasal congestion, mood disorders, sleep disorders, narcolepsy, insomnia, attention deficit disorder (ADD), attention deficit hyperactivity disorder (ADHD), anosmia, hyposmia, mild cognitive impairment (MCI), Down syndrome, Alzheimer's disease, postural reflex abnormality caused by Parkinson's disease, autoimmune autonomic failure, familial dysautonomia, diabetic autonomic neuropathy, amyloidosis in the setting of multiple myeloma, Parkinson's disease, proprandial hypotension, dopamine beta-hydroxylase deficiency, pain, progressive supranuclear palsy, Menkes disease, familial dysautonomia (Riley-Day Syndrome), PD-related dysautonomia (autonomic dysfunction), orthostatic intolerance in adolescents, neurocardiogenic syncope (vasovagal), postural orthostatic tachycardia syndrome (POTS), fibromyalgia, allodynia, hyperalgesia, fatigue, sleep disturbance, depression, chronic orthostatic intolerance, pediatric developmental disorders, genetic diseases involving decreased norepinephrine synthesis or effects, multi-system disorders of regulation, pain, neurodegenerative diseases, cognitive dysfunction, olfactory disorders, neuroendocrine disorders, and autoimmune disorders.

In certain embodiments, neurotransmitter-mediated disorders are selected from the group consisting of dopamine-beta-hydroxylase deficiency, Menkes disease, lack of vitamin C, Lewy body diseases, Parkinson's disease, Lewy body dementia, pure autonomic failure, familial dysautonomia, status-post bilateral endoscopic thoracic sympathectomy, orthostatic intolerance, and orthostatic hypotension.

In certain embodiments, neurotransmitter-mediated disorders are selected from the group consisting of orthostatic hypotension, neurogenic orthostatic hypotension associated with multiple sytem atrophy (MSA), orthostatic hypotension associated with Shy-Drager syndrome, neurogenic orthostatic hypotension associated with familial amyloid polyneuropathy (FAP), neurogenic orthostatic hypotension associated with pure autonomic failure (PAF), idiopathic orthostatic hypotension, asympathicotonic hypotension, neurogenic orthostatic hypotension associated with Parkinson's disease, intradialytic hypotension (IDH), hemodialysis-induced hypotension, hypotension associated with fibromyalgia syndrome (FMS), hypotension in spinal cord injury, and hypotension associated with chronic fatigue syndrome (CFS).

In certain embodiments, neurotransmitter-mediated disorders is orthostatic hypotension.

In certain embodiments, compounds for use in a method of treating a neurotransmitter-mediated disorder comprises administering to the subject a therapeutically effective amount of a compound of as disclosed herein, or a pharmaceutically acceptable salt, solvate, or prodrug thereof, so as to affect: (1) decreased inter-individual variation in plasma levels of the compound or a metabolite thereof; (2) increased average plasma levels of the compound or decreased average plasma levels of at least one metabolite of the compound per dosage unit; (3) decreased inhibition of, and/or metabolism by at least one cytochrome P₄₅₀ or monoamine oxidase isoform in the subject; (4) decreased metabolism via at least one polymorphically-expressed cytochrome P₄₅₀ isoform in the subject; (5) at least one statistically-significantly improved disorder-control and/or disorder-eradication endpoint; (6) an improved clinical effect during the treatment of the disorder, (7) prevention of recurrence, or delay of decline or appearance, of abnormal alimentary or hepatic parameters as the primary clinical benefit, or (8) reduction or elimination of deleterious changes in any diagnostic hepatobiliary function endpoints, as compared to the corresponding non-isotopically enriched compound.

In certain embodiments, inter-individual variation in plasma levels of the compounds as disclosed herein, or metabolites thereof, is decreased; average plasma levels of the compound as disclosed herein are increased; average plasma levels of a metabolite of the compound as disclosed herein are decreased; inhibition of a cytochrome P₄₅₀ or monoamine oxidase isoform by a compound as disclosed herein is decreased; or metabolism of the compound as disclosed herein by at least one polymorphically-expressed cytochrome P₄₅₀ isoform is decreased; by greater than about 5%, greater than about 10%, greater than about 20%, greater than about 30%, greater than about 40%, or by greater than about 50% as compared to the corresponding non-isotopically enriched compound.

Plasma levels of the compound as disclosed herein, or metabolites thereof, may be measured using the methods described by Li et al. Rapid Communications in Mass Spectrometry 2005, 19, 1943-1950, Hughes et al, Xenobiotica 1992, 22(7), 859-69, Varma et al, Journal of Pharmaceutical and Biomedical Analysis 2004, 36(3), 669-674, Massoud et al, Journal of Chromatography, B: Biomedical Sciences and Applications 1999, 734(1), 163-167, Kim et al, Journal of Pharmaceutical and Biomedical Analysis 2003, 31(2), 341-349, and Lindeke et al, Acta Pharmaceutica Suecica 1981, 18(1), 25-34.

Examples of cytochrome P₄₅₀ isoforms in a mammalian subject include, but are not limited to, CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, and CYP51.

Examples of monoamine oxidase isoforms in a mammalian subject include, but are not limited to, MAO_{A}, and MAO_{B}.

The inhibition of the cytochrome P₄₅₀ isoform is measured by the method of Ko et al. (British Journal of Clinical Pharmacology, 2000, 49, 343-351). The inhibition of the MAO_{A} isoform is measured by the method of Weyler et al. (J. Biol Chem. 1985, 260, 13199-13207). The inhibition of the MAO_{B} isoform is measured by the method of Uebelhack et al. (Pharmacopsychiatry, 1998, 31, 187-192).

Examples of polymorphically-expressed cytochrome P₄₅₀ isoforms in a mammalian subject include, but are not limited to, CYP2C8, CYP2C9, CYP2C19, and CYP2D6.

The metabolic activities of liver microsomes, cytochrome P₄₅₀ isoforms, and monoamine oxidase isoforms are measured by the methods described herein.

Examples of improved disorder-control and/or disorder-eradication endpoints, or improved clinical effects include, but are not limited to, blood pressure, mean blood pressure, systolic blood pressure, mean systolic blood pressure, supine blood pressure, mean supine blood pressure, orthostatic systolic BP decrease, Orthostatic Hypotension Questionnaire (OHQ) score, dizziness/lightheadedness score, number of falls, fall-related injuries, Hoehn rating scale score, Yahr rating scale score, visual analog scale (VAS) score, heart rate, forearm vascular resistance, and plasma norepinephrine concentration.

Examples of diagnostic hepatobiliary function endpoints include, but are not limited to, alanine aminotransferase ("ALT"), serum glutamic-pyruvic transaminase ("SGPT"), aspartate aminotransferase ("AST" or "SCOT"), ALT/AST ratios, serum aldolase, alkaline phosphatase ("ALP"), ammonia levels, bilirubin, gamma-glutamyl transpeptidase ("GGTP," "γ-GTP," or "GGT"), leucine aminopeptidase ("LAP"), liver biopsy, liver ultrasonography, liver nuclear scan, 5'-nucleotidase, and blood protein. Hepatobiliary endpoints are compared to the stated normal levels as given in "Diagnostic and Laboratory Test Reference", 4th edition, Mosby, 1999. These assays are run by accredited laboratories according to standard protocol.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

### Combination Therapy

The compounds disclosed herein may also be combined or used in combination with other agents useful in the treatment of tyrosine kinase-mediated disorders. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced).

Such other agents, adjuvants, or drugs, may be administered, by a route and in an amount commonly used therefor, simultaneously or sequentially with a compound as disclosed herein. When a compound as disclosed herein is used contemporaneously with one or more other drugs, a pharmaceutical composition containing such other drugs in addition to the compound disclosed herein may be utilized, but is not required.

In certain embodiments, the compounds disclosed herein can be combined with one or more compounds of structural formula I as disclosed in US Patent No. 7,745,665:

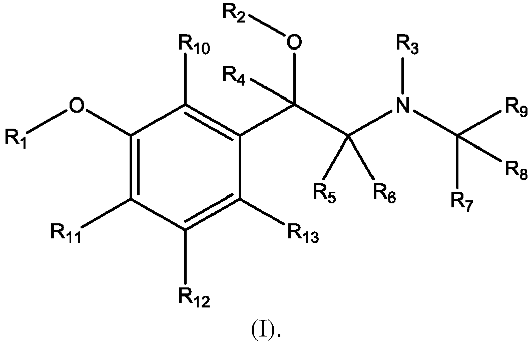

In certain embodiments, the compounds disclosed herein can be combined with a compound having a structural formula selected from the group consisting of 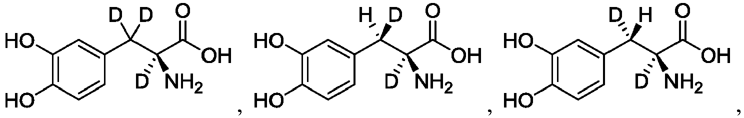 and mixtures thereof. These compounds are disclosed in US Patent No. 8,168,820 and US Patent No. 8,247,603, which are hereby incorporated by reference in their entireties.

In certain embodiments, the compounds disclosed herein can be combined with a mixture of compounds having a structural formula selected from the group consisting of:

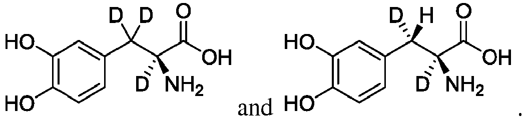

In certain embodiments, the compounds disclosed herein can be combined with a mixture of about 90% of a compound having the structural formula: 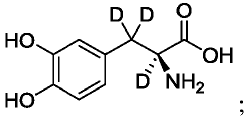 and
about 10% of a compound having the structural formula:

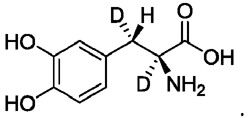

In certain embodiments, the compounds disclosed herein can be combined with one or more sympathomimetic agents selected from the group consisting of epinephrine, norepinephrine, phenylephrine, dobutamine, dopamine, ephedrine, midodrine, and amezinium.

In certain embodiments, the compounds disclosed herein can be combined with one or more S-alkylisothiouronium derivatives selected from the group consisting of difetur and izoturon.

In certain embodiments, the compounds disclosed herein can be combined with one or more glucocorticoids selected from the group consisting of hydrocortisone, prednisone, prednisolone, dexamethasone, and betamethasone.

In certain embodiments, the compounds disclosed herein can be combined with one or more analeptics selected from the group consisting of bemegride, caffeine, camphora, and cordiamine.

In certain embodiments, the compounds disclosed herein can be combined with one or more psychotropics selected from the group consisting of amphetamine, atomoxetine, bupropion, duloxetine, methamphetamine, methylphenidate, reboxetine, and venlafaxine.

In certain embodiments, the compounds disclosed herein can be combined with one or more positive inotropic agents selected from the group consisting of cardiac glycosides, strophantin K, corglycon, digoxin, amrinone, and milrinone.

In certain embodiments, the compounds disclosed herein can be combined with one or more antihypotensive agents selected from the group consisting of angiotensinamide, indomethacin, oxilofrine, potassium chloride, and yohimbine.

In certain embodiments, the compounds disclosed herein can be combined with one or more L-aromatic-amino acid decarboxylase inhibitor selected from the group consisting of benserazide, carbidopa, methyldopa, and α-difluoromethyl-DOPA.

In certain embodiments, the compounds disclosed herein can be combined with one or more catechol-O-methyltransferase inhibitors selected from the group consisting of entacapone, tolcapone, and nitecapone.

In certain embodiments, the compounds disclosed herein can be combined with one or more monoamine oxidase inhibitors selected from the group consisting of isocarboxazid, isoniazid, nialamide, phenelzine, tranylcypromine, moclobemide, pirlindole, toloxatone, rasagiline, and selegiline.

In certain embodiments, the compounds disclosed herein can be combined with one or more 5-HT_{2A} inverse agonist selected from the group consisting of pimvaserin.

The compounds disclosed herein can also be administered in combination with other classes of compounds, including, but not limited to, norepinephrine reuptake inhibitors (NRIs) such as atomoxetine; dopamine reuptake inhibitors (DARIs), such as methylphenidate; serotonin-norepinephrine reuptake inhibitors (SNRIs), such as milnacipran; sedatives, such as diazepham; norepinephrine-dopamine reuptake inhibitor (NDRIs), such as bupropion; serotonin-norepinephrine-dopamine-reuptake-inhibitors (SNDRIs), such as venlafaxine; monoamine oxidase inhibitors, such as selegiline; hypothalamic phospholipids; endothelin converting enzyme (ECE) inhibitors, such as phosphoramidon; opioids, such as tramadol; thromboxane receptor antagonists, such as ifetroban; potassium channel openers; thrombin inhibitors, such as hirudin; hypothalamic phospholipids; growth factor inhibitors, such as modulators of PDGF activity; platelet activating factor (PAF) antagonists; anti-platelet agents, such as GPIIb/IIIa blockers (e.g., abdximab, eptifibatide, and tirofiban), P2Y(AC) antagonists (e.g., clopidogrel, ticlopidine and CS-747), and aspirin; anticoagulants, such as warfarin; low molecular weight heparins, such as enoxaparin; Factor VIIa Inhibitors and Factor Xa Inhibitors; renin inhibitors; neutral endopeptidase (NEP) inhibitors; vasopepsidase inhibitors (dual NEP-ACE inhibitors), such as omapatrilat and gemopatrilat; HMG CoA reductase inhibitors, such as pravastatin, lovastatin, atorvastatin, simvastatin, NK-104 (a.k.a. itavastatin, nisvastatin, or nisbastatin), and ZD-4522 (also known as rosuvastatin, or atavastatin or visastatin); squalene synthetase inhibitors; fibrates; bile acid sequestrants, such as questran; niacin; anti-atherosclerotic agents, such as ACAT inhibitors; MTP Inhibitors; calcium channel blockers, such as amlodipine besylate; potassium channel activators; alpha-muscarinic agents; beta-muscarinic agents, such as carvedilol and metoprolol; antiarrhythmic agents; diuretics, such as chlorothlazide, hydrochiorothiazide, flumethiazide, hydroflumethiazide, bendroflumethiazide, methylchlorothiazide, trichioromethiazide, polythiazide, benzothlazide, ethacrynic acid, tricrynafen, chlorthalidone, furosenilde, musolimine, bumetanide, triamterene, amiloride, and spironolactone; thrombolytic agents, such as tissue plasminogen activator (tPA), recombinant tPA, streptokinase, urokinase, prourokinase, and anisoylated plasminogen streptokinase activator complex (APSAC); anti-diabetic agents, such as biguanides (e.g. metformin), glucosidase inhibitors (e.g., acarbose), insulins, meglitinides (e.g., repaglinide), sulfonylureas (e.g., glimepiride, glyburide, and glipizide), thiozolidinediones (e.g. troglitazone, rosiglitazone and pioglitazone), and PPAR-gamma agonists; mineralocorticoid receptor antagonists, such as spironolactone and eplerenone; growth hormone secretagogues; aP2 inhibitors; phosphodiesterase inhibitors, such as PDE III inhibitors (e.g., cilostazol) and PDE V inhibitors (e.g., sildenafil, tadalafil, vardenafil); protein tyrosine kinase inhibitors; antiinflammatories; antiproliferatives, such as methotrexate, FK506 (tacrolimus, Prograf), mycophenolate mofetil; chemotherapeutic agents; immunosuppressants; anticancer agents and cytotoxic agents (e.g., alkylating agents, such as nitrogen mustards, alkyl sulfonates, nitrosoureas, ethylenimines, and triazenes); antimetabolites, such as folate antagonists, purine analogues, and pyrridine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (e.g., cortisone), estrogens/antiestrogens, androgens/antiandrogens, progestins, and luteinizing hormone-releasing hormone anatagonists, and octreotide acetate; microtubule-disruptor agents, such as ecteinascidins; microtubule-stablizing agents, such as pacitaxel, docetaxel, and epothilones A-F; plant-derived products, such as vinca alkaloids, epipodophyllotoxins, and taxanes; and topoisomerase inhibitors; prenyl-protein transferase inhibitors; and cyclosporins; steroids, such as prednisone and dexamethasone; cytotoxic drugs, such as azathiprine and cyclophosphamide; TNF-alpha inhibitors, such as tenidap; anti-TNF antibodies or soluble TNF receptor, such as etanercept, rapamycin, and leflunimide; and cyclooxygenase-2 (COX-2) inhibitors, such as celecoxib and rofecoxib; and miscellaneous agents such as, hydroxyurea, procarbazine, mitotane, hexamethylmelamine, gold compounds, platinum coordination complexes, such as cisplatin, satraplatin, and carboplatin.

### General Synthetic Methods for Preparing Compounds

Isotopic hydrogen can be introduced into a compound as disclosed herein by synthetic techniques that employ deuterated reagents, whereby incorporation rates are pre-determined; and/or by exchange techniques, wherein incorporation rates are determined by equilibrium conditions, and may be highly variable depending on the reaction conditions. Synthetic techniques, where tritium or deuterium is directly and specifically inserted by tritiated or deuterated reagents of known isotopic content, may yield high tritium or deuterium abundance, but can be limited by the chemistry required. Exchange techniques, on the other hand, may yield lower tritium or deuterium incorporation, often with the isotope being distributed over many sites on the molecule.

The compounds as disclosed herein can be prepared by methods known to one of skill in the art and routine modifications thereof, and/or following procedures similar to those described in the Example section herein and routine modifications thereof, and/or procedures found in EP 84928 B1, EP 128684 A1, DE 19619510 A1, JP 1997249626 A, WO 2011001976 A1, and WO 2013142093 A1, which are hereby incorporated in their entirety, and references cited therein and routine modifications thereof. Compounds as disclosed herein can also be prepared as shown in any of the following schemes and routine modifications thereof.

The following schemes can be used to practice the present invention. Any position shown as hydrogen may optionally be replaced with deuterium.

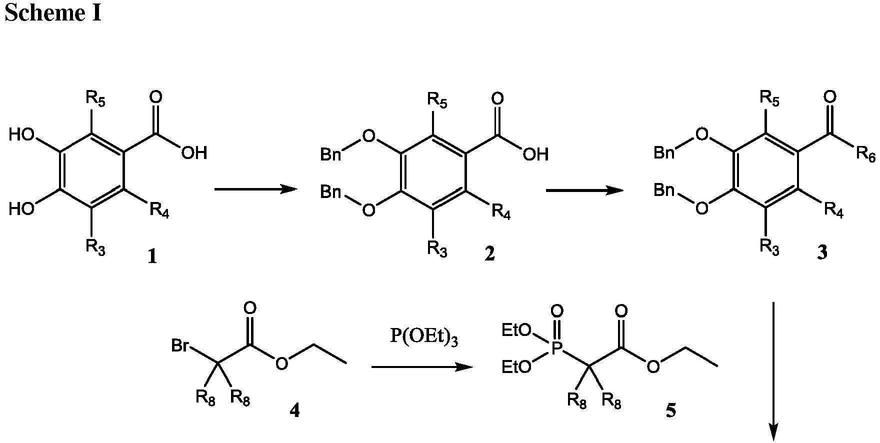

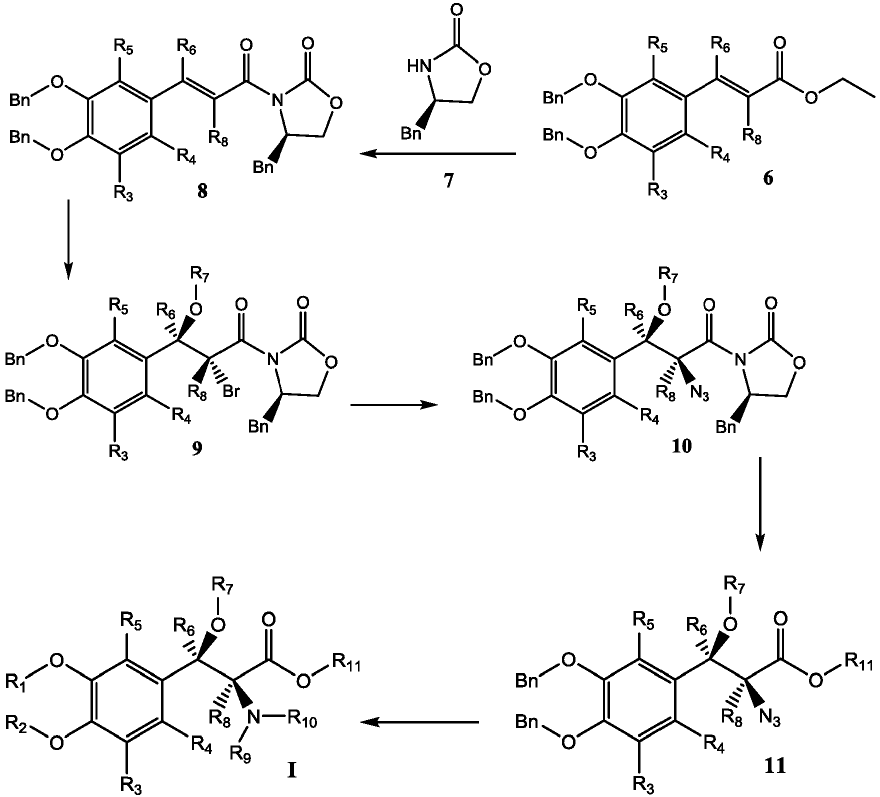

Compound **1** is reacted with an appropriate protecting agent, such as benzyl chloride to give compound **2.** Compound **2** is treated with an appropriate chlorinating agent, such as thionyl chloride, followed by an appropriate reducing agent, such as a combination of palladium on barium sulfate and hydrogen, to give compound **3.** Compound **4** is reacted with triethyl phosphate to give compound **5.** Compound **3** is reacted with compound **5,** in the presence of an appropriate base, such as sodium hydride, to give compound **6.** Compound **6** is reacted with compound **7,** in the presence of an appropriate base, such as potassium hydoxide, to give compound **8.** Compound **8** is reacted with an appropriate oxidizing agent, such sodium periodate, and an appropriate bromide salt, such as lithium bromide, to give compound **9.** Compound **9** is reacted with sodium azide to give compound **10.** Compound **10** is reacted with an appropriate oxazolidinone deprotecting agent, such as a mixture of lithium hydroxide and hydrogen peroxide, to give compound **11.** Compound **11** is reacted with an appropriate reducing agent, such as a combination of palladium on carbon and hydrogen, to give a compound of formula **I.** The hydrochloride salt of the compound of formula **I** can be prepared by reacting the compound of formula **I** with hydrochloric acid in an appropriate solvent, such as a mixture of water and isopropanol.

Deuterium can be incorporated to different positions synthetically, according to the synthetic procedures as shown in Scheme **I,** by using appropriate deuterated intermediates. For example, to introduce deuterium at one or more positions of R₃-R₅, compound **1** with the corresponding deuterium substitutions can be used. To introduce deuterium at R₆, deuterium gas can be used. To introduce deuterium at R₈, compound **4** with the corresponding deuterium substitutions can be used.

Deuterium can be incorporated to various positions having an exchangeable proton, such as the phenyl hydroxyl O-Hs, the benzylic alcohol hydroxyl O-H, the amine N-Hs, and the carboxyl O-H, via proton-deuterium equilibrium exchange. For example, to introduce deuterium at R₁-R₂, R₇, R₉-R₁₀, and R₁₁, these protons may be replaced with deuterium selectively or non-selectively through a proton-deuterium exchange method known in the art.

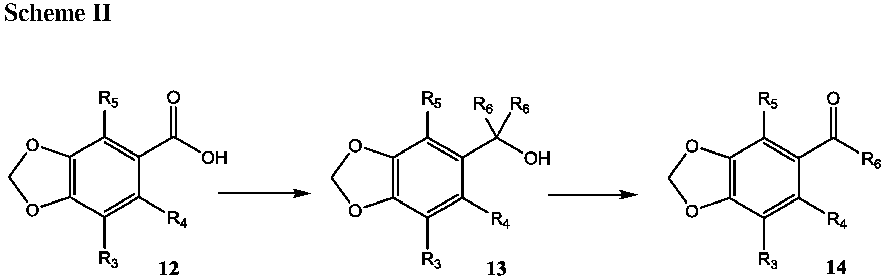

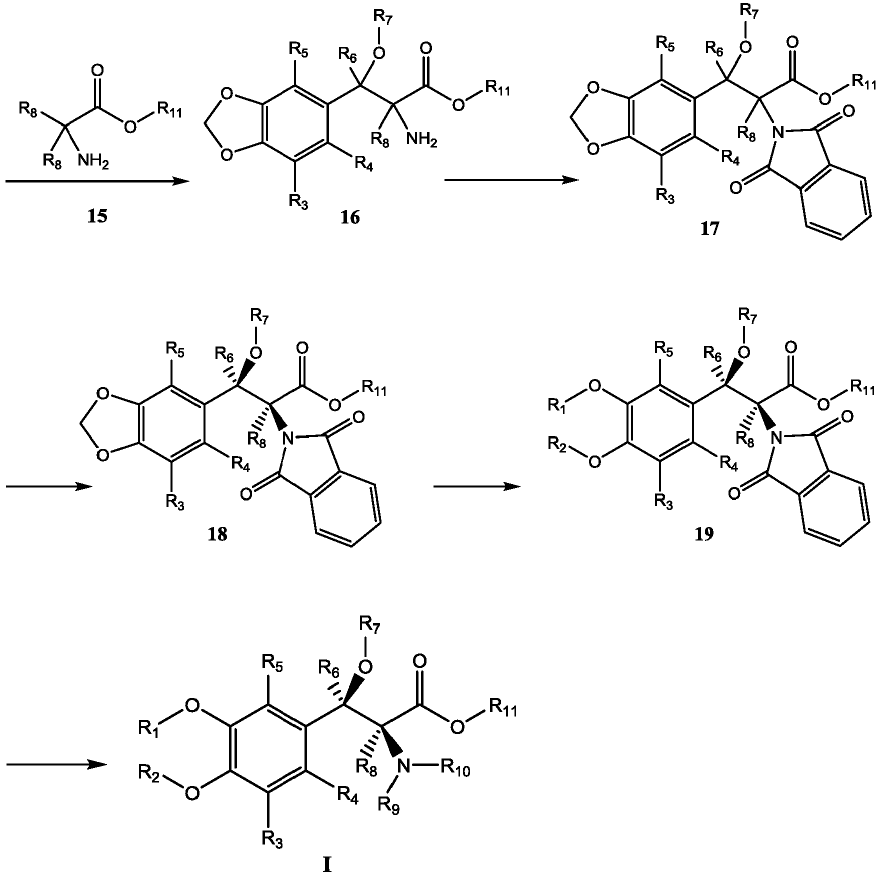

Compound **12** is reacted with an appropriate reducing agent, such as lithium aluminum hydride, in an appropriate solvent, such as tetrahydrofuran, to give compound **13.** Compound **13** is treated with an appropriate oxidizing agent, such as Dess-Martin periodinane, in an appropriate solvent, such as dichloromethane, to give compound **14.** Compound **14** is reacted with compound **15,** in the presence of an appropriate base, such as potassium hydroxide, in an appropriate solvent, such as a mixture of toluene and methanol, to give compound **16.** Compound **16** is reacted with an appropriate amine protecting reagent, such as N-carbomethoxy pthalimide, in an appropriate solvent, such as water, in the presence of an appropriate base, such as sodium carbonate, then reacted with an appropriate acid, such as sulfuric acid, to give compound **17.** Compound **17** is reacted with an appropriate chiral resolving agent, such as L-norephedrine, in an appropriate solvent, such as methanol, to give the L-norephedrine salt of compound **18**, which is further treated with an appropriate acid, such as sulfuric acid, in an appropriate solvent, such as water, to give compound **18** as the free acid. Compound **18** is reacted with an appropriate methylenedioxy deprotecting agent, such as a mixture of aluminum chloride and octanethiol, in an appropriate solvent, such as dichloromethane, to give compound **19.** Compound **19** is reacted with an appropriate pthalimide deprotecting agent, such as a mixture of hydroxylamine hydrochloride and sodium bicarbonate, in an appropriate solvent, such as methanol, at an elevated temperature, to give a compound of formula **I.** The hydrochloride salt of the compound of formula **I** can be prepared by reacting the compound of formula **I** with hydrochloric acid in an appropriate solvent, such as a mixture of water and isopropanol.

Deuterium can be incorporated to different positions synthetically, according to the synthetic procedures as shown in Scheme **I,** by using appropriate deuterated intermediates. For example, to introduce deuterium at one or more positions of R₃-R₅, compound **12** with the corresponding deuterium substitutions can be used. To introduce deuterium at R₆, lithium aluminum deuteride can be used. To introduce deuterium at R₈, compound **15** with the corresponding deuterium substitutions can be used.

Deuterium can be incorporated to various positions having an exchangeable proton, such as the phenyl hydroxyl O-Hs, the benzylic alcohol hydroxyl O-H, the amine N-Hs, and the carboxyl O-H, via proton-deuterium equilibrium exchange. For example, to introduce deuterium at R₁-R₂, R₇, R₉-R₁₀, and R₁₁, these protons may be replaced with deuterium selectively or non-selectively through a proton-deuterium exchange method known in the art.

The following compounds can generally be made using the methods described above. Only the compounds falling under claim 1 are part of the invention. The remaining compounds may serve as reference compounds. 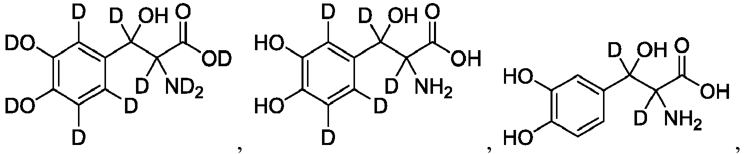 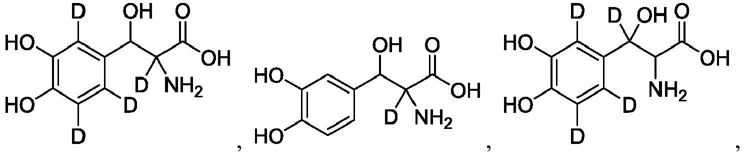 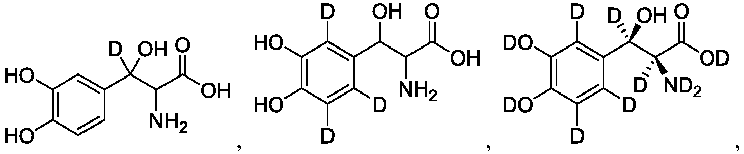 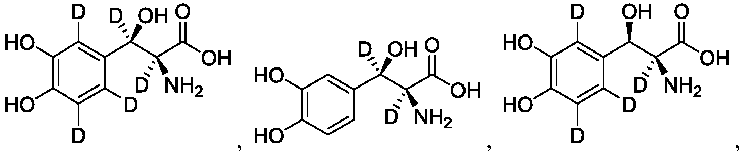 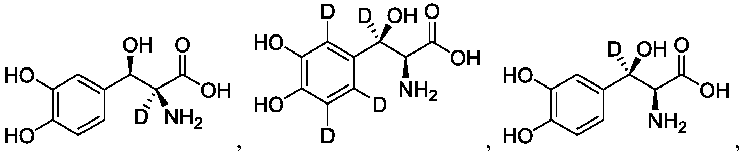 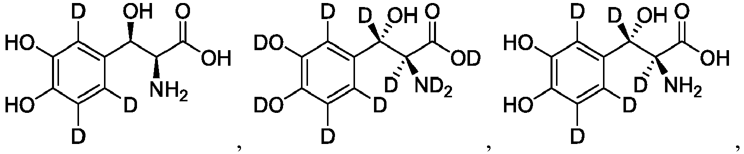 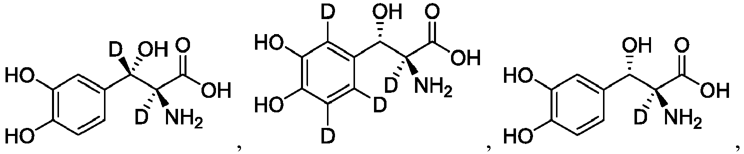 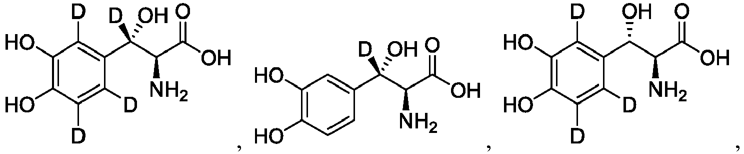 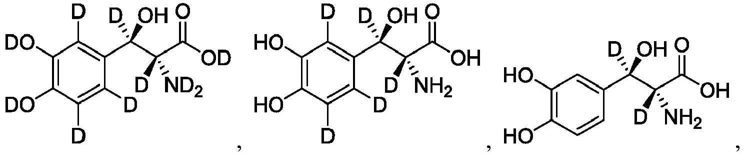 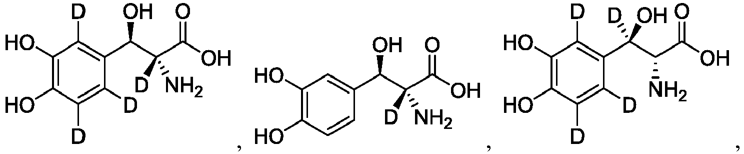 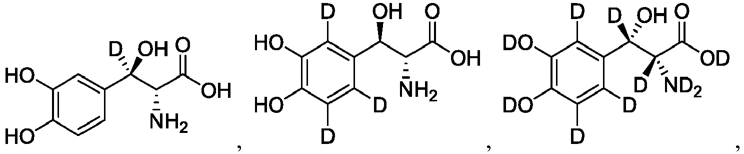 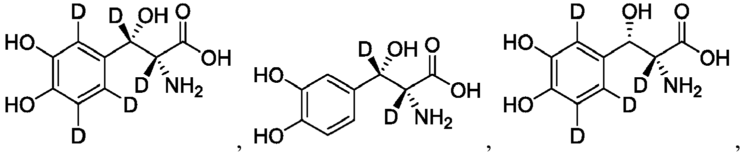 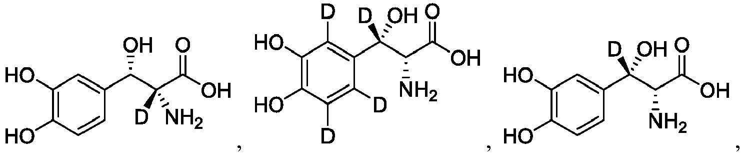 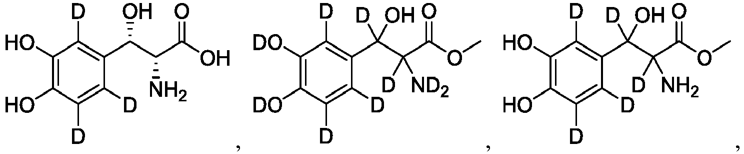 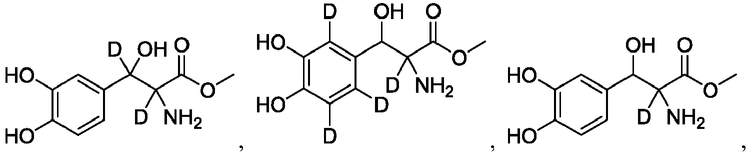 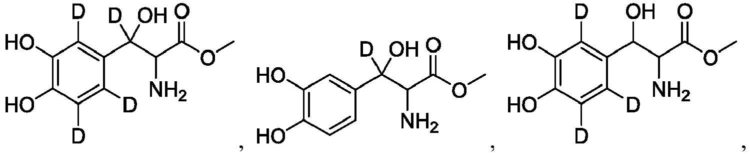 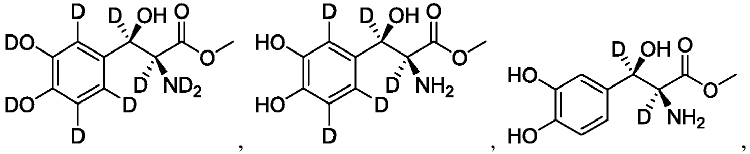 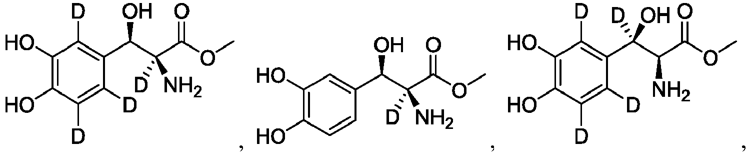 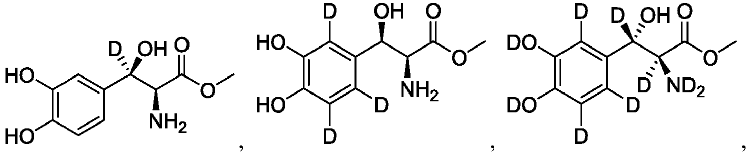 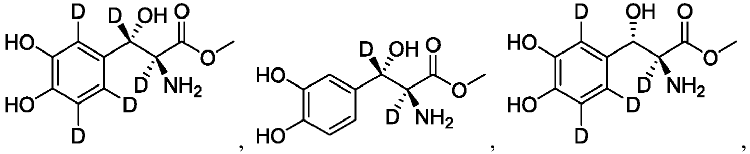 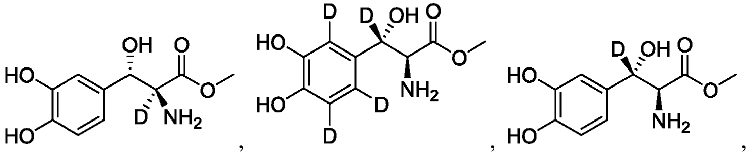 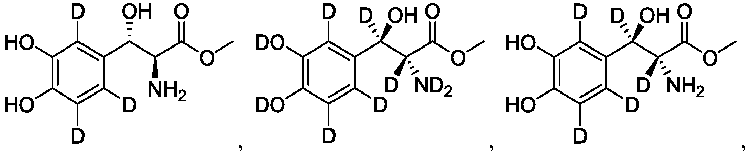 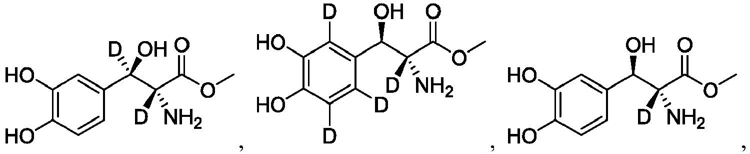 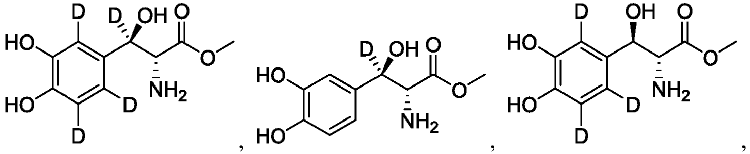 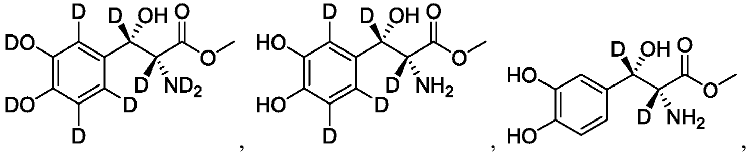 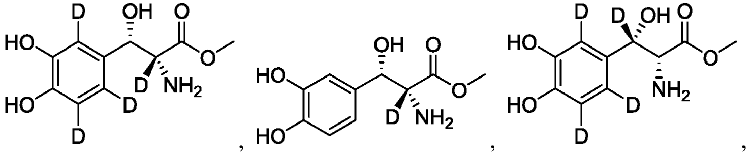 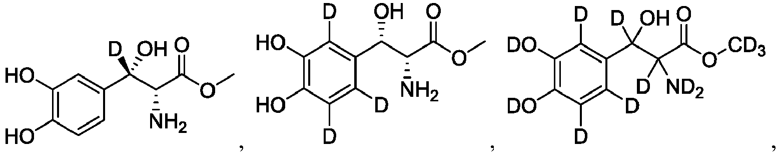 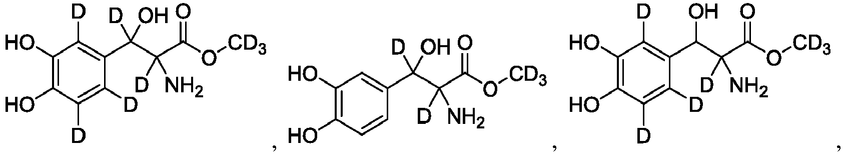 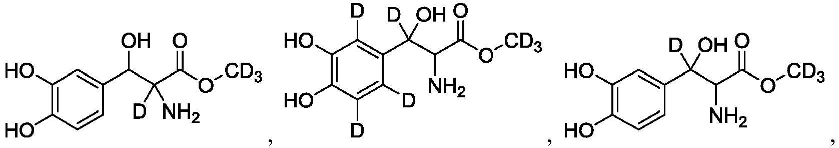 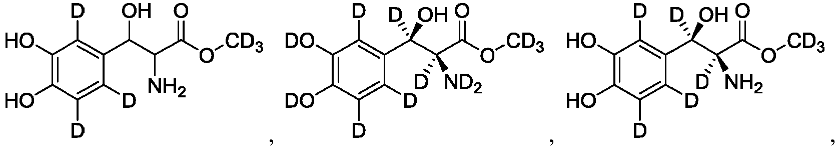 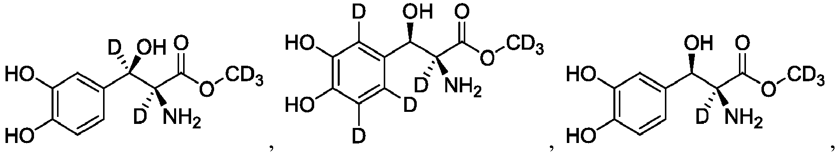 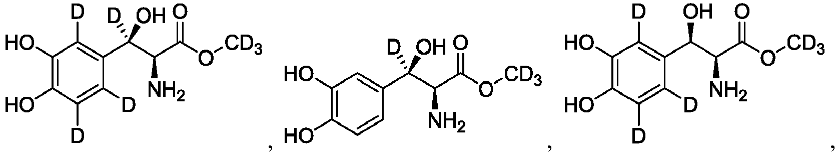 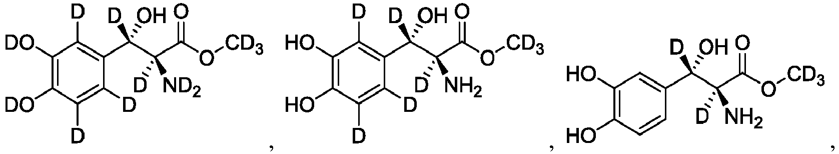 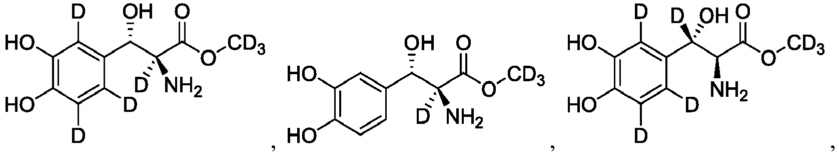 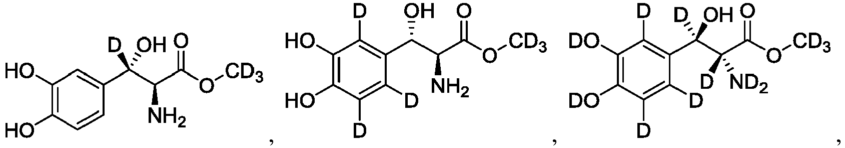 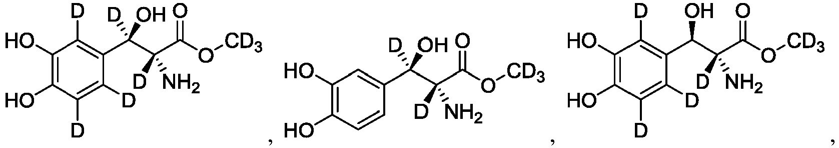 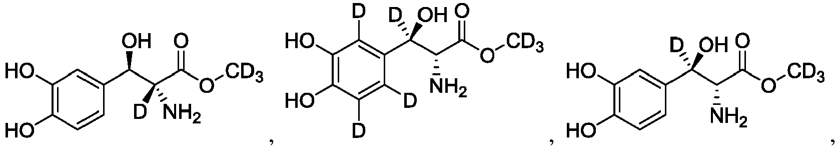 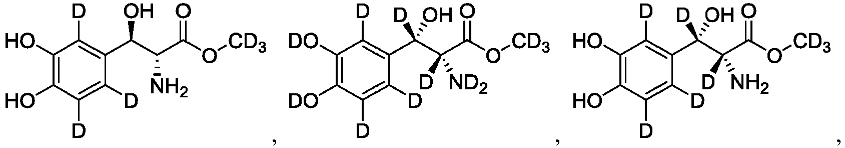 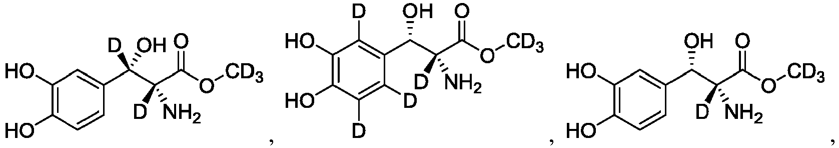 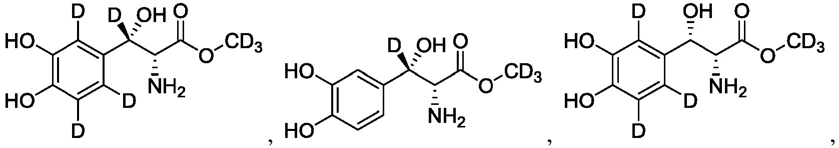 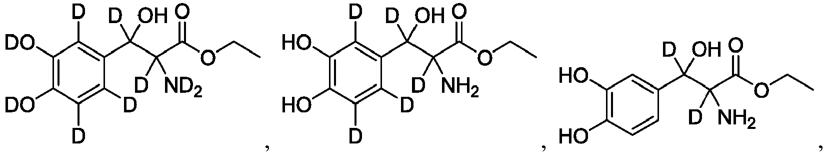 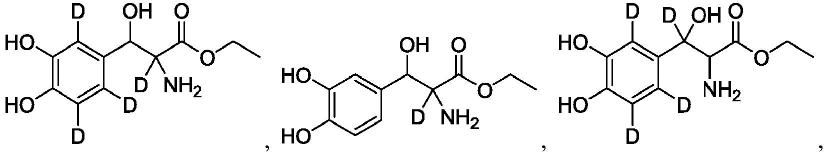 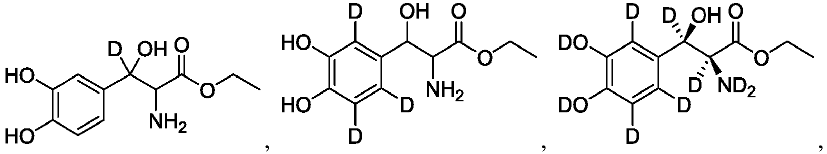 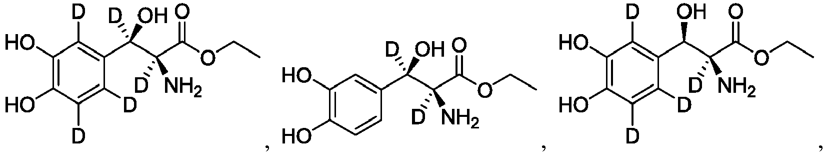 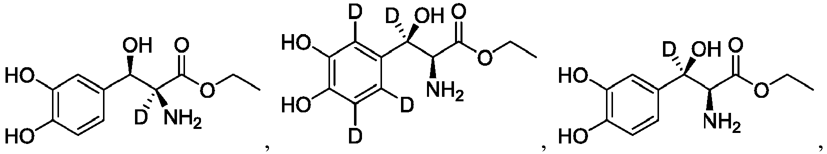 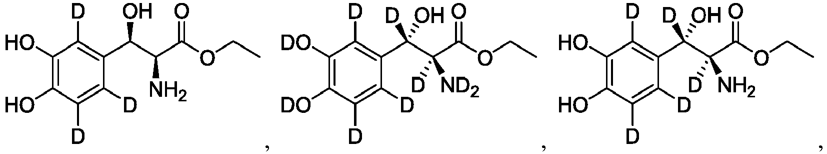 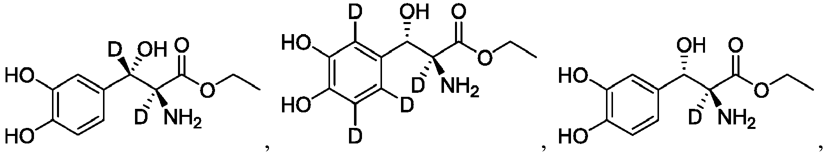 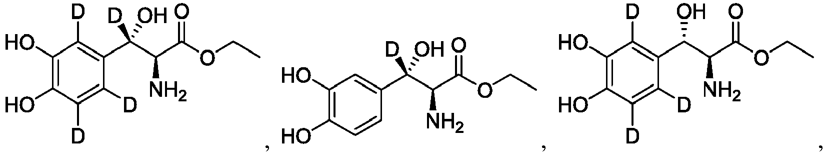 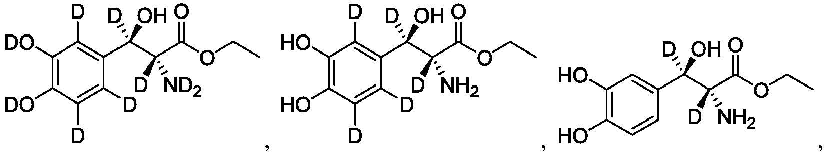 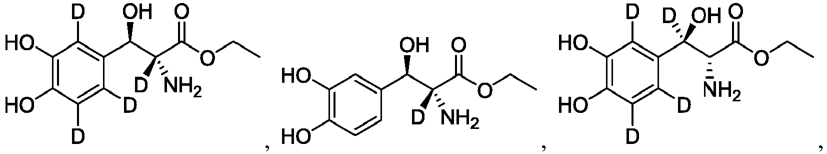 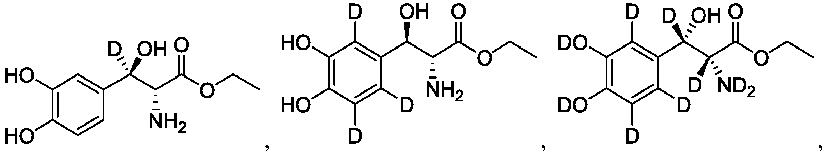 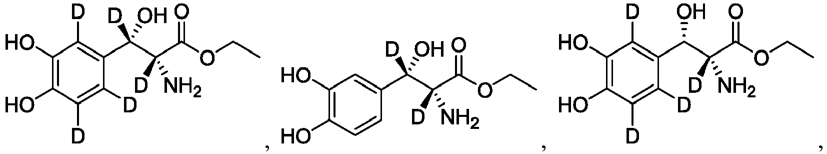 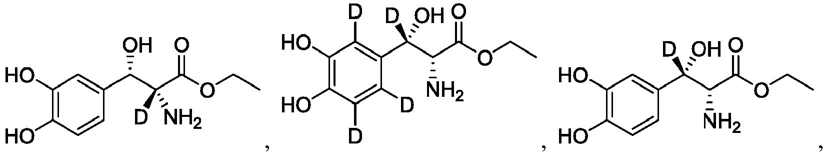 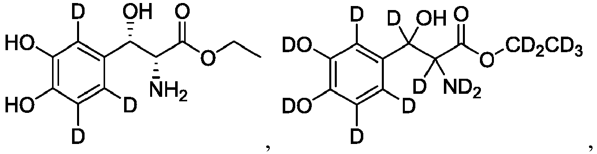 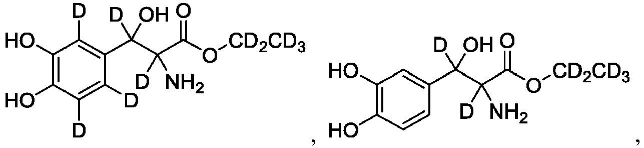 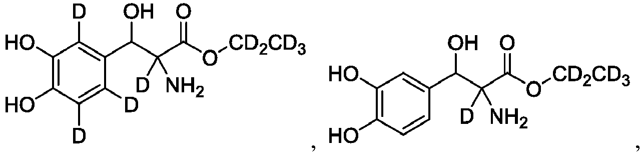 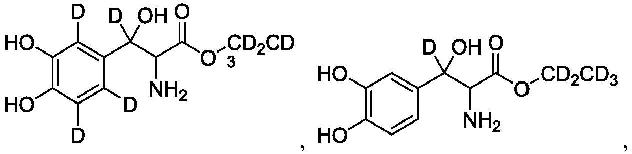 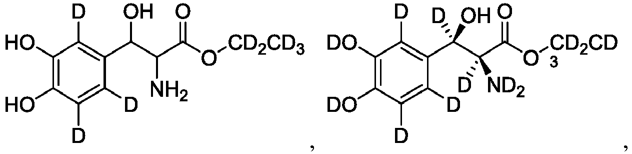 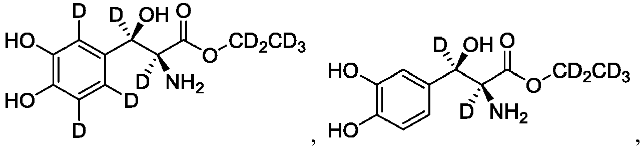 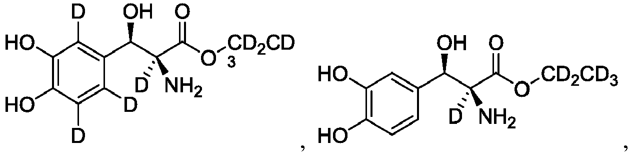 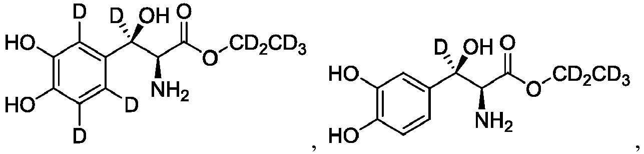 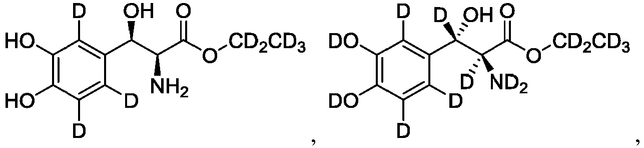 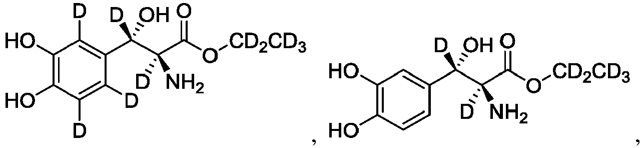 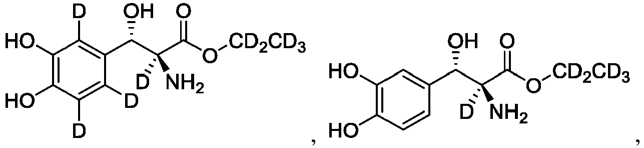 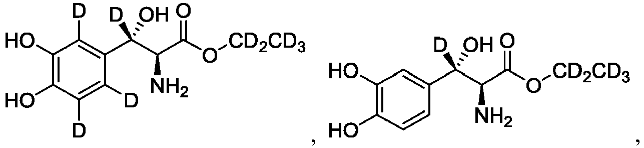 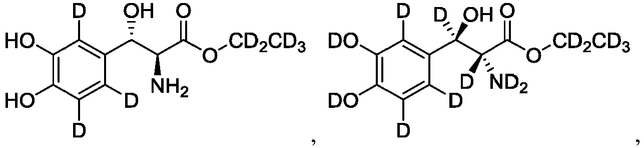 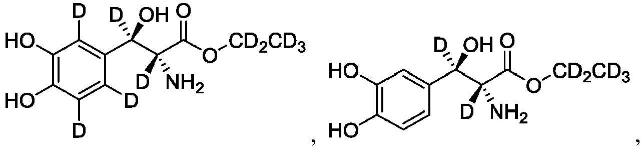 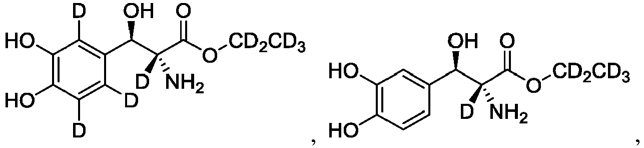 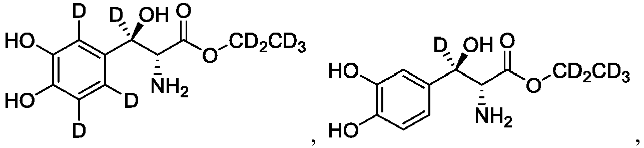 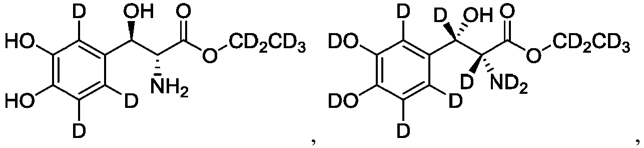 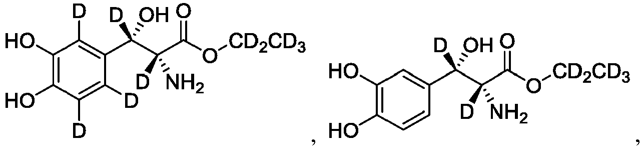 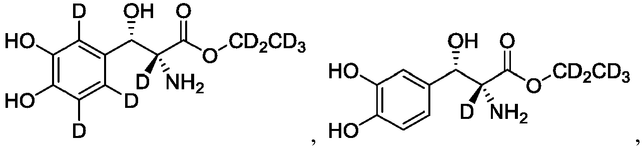 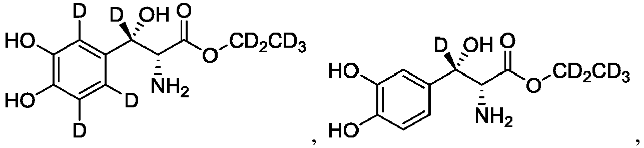 and

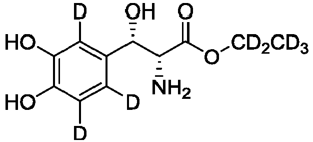

Changes in the metabolic properties of the compounds disclosed herein as compared to their non-isotopically enriched analogs can be shown using the following assays.

### Biological Activity Assays

### Change of mean arterial blood pressure in anesthetized rats following intravenous administration of 2 mg/kg L-threo-2,3-dideutero POPS in comparison to the same dose of L-threo-DOPS

The administration of L-threo-2,3-dideutero DOPS leads to an enhanced and prolonged increase of the mean arterial blood pressure.

### In vitro Liver Microsomal Stability Assay

Liver microsomal stability assays are conducted at 1 mg per mL liver microsome protein with an NADPH-generating system in 2% NaHCO₃ (2.2 mM NADPH, 25.6 mM glucose 6-phosphate, 6 units per mL glucose 6-phosphate dehydrogenase and 3.3 mM MgCl₂). Test compounds are prepared as solutions in 20% acetonitrile-water and added to the assay mixture (final assay concentration 5 microgram per mL) and incubated at 37 °C. Final concentration of acetonitrile in the assay should be <1%. Aliquots (50µL) are taken out at times 0, 15, 30, 45, and 60 min, and diluted with ice cold acetonitrile (200 µL) to stop the reactions. Samples are centrifuged at 12,000 RPM for 10 min to precipitate proteins. Supernatants are transferred to microcentrifuge tubes and stored for LC/MS/MS analysis of the degradation half-life of the test compounds.

### In vitro Monoamine Oxidase A Degradation Assay

Norepinephrine and and d₆-norepinephrine were incubated with monoamine oxidase-A (MAO-A). 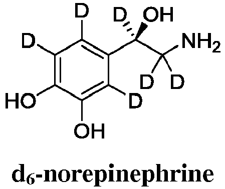 The appearance of 3,4-dihydroxyphenylglycolaldehyde and the disappearance of norepinephrine were tracked. Compared to non-deuterated norepinephrine, d₆-norepinephrine was associated with about a 5-fold decrease in digestion by MAO-A and about a 75% decrease in 3,4-dihydroxyphenylglycolaldehyde production.

The assay method is a batch alumina extraction followed by liquid chromatography with electrochemical detection. The post-column electrodes are arranged in series, with an oxidizing potential at the first electrode and reducing potential at the third. This series arrangement of flow-through electrodes reduces the solvent front substantially and improves the sensitivity and specificity for detecting reversibly oxidized species such as catechols. 3,4-Dihydroxyphenylglycolaldehyde is identified by a broad, short peak within the solvent front..

### In vitro metabolism using human cytochrome P₄₅₀ enzymes

The cytochrome P₄₅₀ enzymes are expressed from the corresponding human cDNA using a baculovirus expression system (BD Biosciences, San Jose, CA). A 0.25 milliliter reaction mixture containing 0.8 milligrams per milliliter protein, 1.3 millimolar NADP⁺, 3.3 millimolar glucose-6-phosphate, 0.4 U/mL glucose-6-phosphate dehydrogenase, 3.3 millimolar magnesium chloride and 0.2 millimolar of a compound of Formula I, the corresponding non-isotopically enriched compound or standard or control in 100 millimolar potassium phosphate (pH 7.4) is incubated at 37 °C for 20 min. After incubation, the reaction is stopped by the addition of an appropriate solvent (e.g., acetonitrile, 20% trichloroacetic acid, 94% acetonitrile/6% glacial acetic acid, 70% perchloric acid, 94% acetonitrile/6% glacial acetic acid) and centrifuged (10,000 g) for 3 min. The supernatant is analyzed by HPLC/MS/MS.

| **Cytochrome P₄₅₀** | **Standard** |
|---|---|
| CYP1A2 | Phenacetin |
| CYP2A6 | Coumarin |
| CYP2B6 | [¹³C]-(S)-mephenytoin |
| CYP2C8 | Paclitaxel |
| CYP2C9 | Diclofenac |
| CYP2C19 | [¹³C]-(S)-mephenytoin |
| CYP2D6 | (+/-)-Bufuralol |
| CYP2E1 | Chlorzoxazone |
| CYP3A4 | Testosterone |
| CYP4A | [¹³C]-Lauric acid |

### Monoamine Oxidase A Inhibition and Oxidative Turnover

The procedure is carried out using the methods described by Weyler, Journal of Biological Chemistry 1985, 260, 13199-13207, which is hereby incorporated by reference in its entirety. Monoamine oxidase A activity is measured spectrophotometrically by monitoring the increase in absorbance at 314 nm on oxidation of kynuramine with formation of 4-hydroxyquinoline. The measurements are carried out, at 30 °C, in 50mM NaP; buffer, pH 7.2, containing 0.2% Triton X-100 (monoamine oxidase assay buffer), plus 1 mM kynuramine, and the desired amount of enzyme in 1 mL total volume.

### Monooamine Oxidase B Inhibition and Oxidative Turnover

The procedure is carried out as described in Uebelhack, Pharmacopsychiatry 1998, 31(5), 187-192.

### In Vitro Rat CNS Extracellular Norepinephrine Production

The procedure is carried out as described in Verhagen-Kamerbeek et al., Monit. Mol. Neurosci., Proc. Int. Conf. In Vivo Methods, 5th, 1991, 373-6.

### Endogenous Norepinephrine Release From Presynaptic Receptors in Rat Hypothalamic Slices

The procedure is carried out as described in Yue et al., J. Pharmacy and Pharmacol., 1992, 44(12), 990-5.

### Hemodynamic and Renal Alterations of Portal Hypertensive Rats

The procedure is carried out as described in Coll Mar et al., Hepatology (Baltimore, Md.), 2012, 56(5), 1849-60.

## Claims

1. A deuterium-enriched compound that is:

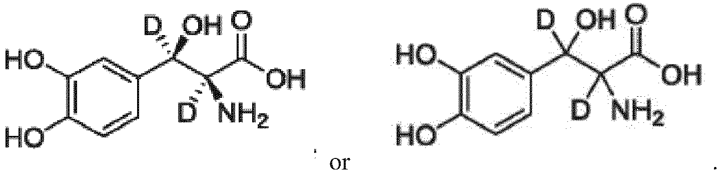

2. The compound of claim 1, that is:

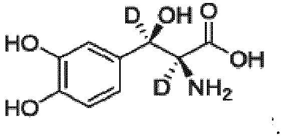

3. The compound of claim 1 or claim 2, wherein each position represented as D has deuterium enrichment of no less than 10%.

4. The compound of any one of the preceding claims, wherein each position represented as D has a deuterium enrichment of no less than 50%.

5. The compound of any one of the preceding claims, wherein each position represented as D has a deuterium enrichment of no less than 90%.

6. The compound of any one of the preceding claims, wherein each position represented as D has a deuterium enrichment of no less than 98%.

7. A pharmaceutical composition comprising a pharmaceutically acceptable carrier together with a compound of any one of the preceding claims.

8. A compound of any one of claims 1 to 6, or the pharmaceutical composition of claim 7, for use as a medicament.

9. A compound of any one of claims 1 to 6, or the pharmaceutical composition of claim 7, for use in the prevention or treatment of a disorder ameliorated by modulating neurotransmitter levels, wherein said disorder is selected from the group consisting of: hypotension, orthostatic hypotension, neurogenic orthostatic hypotension, symptomatic neurogenic orthostatic hypotension, neurogenic orthostatic hypotension associated with multiple system atrophy (MSA), orthostatic hypotension associated with Shy-Drager syndrome, neurogenic orthostatic hypotension associated with familial amyloid polyneuropathy (FAP), neurogenic orthostatic hypotension associated with pure autonomic failure (PAF) , idiopathic orthostatic hypotension, asympathicotonic hypotension, neurogenic orthostatic hypotension associated with Parkinson's disease, intradialytic hypotension (IDH), hemodialysis-induced hypotension, hypotension associated with fibromyalgia syndrome (FMS), hypotension in spinal cord injury, hypotension associated with chronic fatigue syndrome (CFS), frozen gait, akinesia, and dysarthria in Parkinson's disease, Lewy body dementia, rapid eye movement (REM) behavior disorder, chronic heart failure, stress-related disorders, motor or speech disturbances, chronic pain, stroke, cerebral ischemia, nasal congestion, mood disorders, sleep disorders, narcolepsy, insomnia, attention deficit disorder (ADD), attention deficit hyperactivity disorder (ADHD), anosmia, hyposmia, mild cognitive impairment (MCI), Down syndrome, Alzheimer's disease, postural reflex abnormality caused by Parkinson's disease, autoimmune autonomic failure, familial dysautonomia, diabetic autonomic neuropathy, amyloidosis in the setting of multiple myeloma, Parkinson's disease, proprandial hypotension, dopamine beta-hydroxylase deficiency, pain, progressive supranuclear palsy, Menkes disease, familial dysautonomia (Riley-Day Syndrome), PD-related dysautonomia (autonomic dysfunction), orthostatic intolerance in adolescents, neurocardiogenic syncope (vasovagal), postural orthostatic tachycardia syndrome (POTS), fibromyalgia, allodynia, hyperalgesia, fatigue, sleep disturbance, depression, chronic orthostatic intolerance, pediatric developmental disorders, genetic diseases involving decreased norepinephrine synthesis or effects, multi-system disorders of regulation, pain, neurodegenerative diseases, cognitive dysfunction, olfactory disorders, neuroendocrine disorders, and autoimmune disorders; preferably wherein said disorder is selected from the group consisting of orthostatic hypotension, neurogenic orthostatic hypotension associated with multiple system atrophy (MSA), orthostatic hypotension associated with Shy-Drager syndrome, neurogenic orthostatic hypotension associated with familial amyloid polyneuropathy (FAP), neurogenic orthostatic hypotension associated with pure autonomic failure (PAF) idiopathic orthostatic hypotension, asympathicotonic hypotension, neurogenic orthostatic hypotension associated with Parkinson's disease, intradialytic hypotension (IDH), hemodialysis-induced hypotension, hypotension associated with fibromyalgia syndrome (FMS), hypotension in spinal cord injury, and hypotension associated with chronic fatigue syndrome (CFS); and most preferably wherein said disorder is orthostatic hypotension.

10. A compound according to any one of claims 1 to 6, or the pharmaceutical composition of claim 7, for use in the prevention or treatment of a disorder ameliorated by modulating neurotransmitter levels, wherein said disorder is selected from the group consisting of dopamine-beta-hydroxylase deficiency, Menkes disease, lack of vitamin C, Lewy body diseases, Parkinson's disease, Lewy body dementia, pure autonomic failure, familial dysautonomia, status-post bilateral endoscopic thoracic sympathectomy, orthostatic intolerance, and orthostatic hypotension.

11. The compound of any of claims 1-6, or the pharmaceutical composition of claim 7, for use according to claim 9 or claim 10, wherein the compound or pharmaceutical composition is used in combination with an additional therapeutic agent.

12. The compound of any of claims 1-6, or the pharmaceutical composition of claim 7, for use according to claim 11, wherein the additional therapeutic agent is selected from the group consisting of: 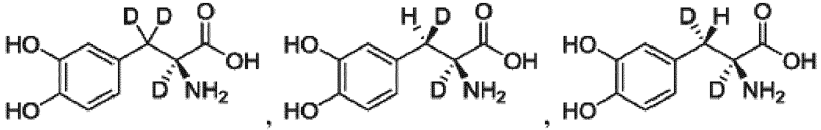 and mixtures thereof.

## Patentansprüche

1. Mit Deuterium angereicherte Verbindung mit folgenden Formeln:

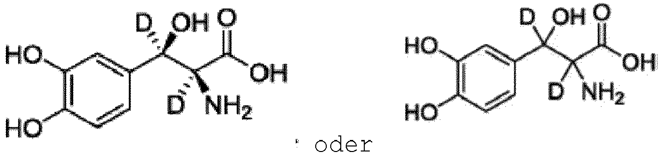

2. Verbindung nach Anspruch 1, und zwar:

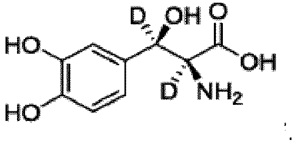

3. Verbindung nach Anspruch 1 oder Anspruch 2, wobei jede als D dargestellte Position eine Deuteriumanreicherung von mindestens 10 % aufweist.

4. Verbindung nach einem der vorstehenden Ansprüche, wobei jede als D dargestellte Position eine Deuteriumanreicherung von mindestens 50 % aufweist.

5. Verbindung nach einem der vorstehenden Ansprüche, wobei jede als D dargestellte Position eine Deuteriumanreicherung von mindestens 90 % aufweist.

6. Verbindung nach einem der vorstehenden Ansprüche, wobei jede als D dargestellte Position eine Deuteriumanreicherung von mindestens 98 % aufweist.

7. Pharmazeutische Zusammensetzung, umfassend ein pharmazeutisch unbedenkliches Trägermaterial zusammen mit einer Verbindung nach einem der vorstehenden Ansprüche.

8. Verbindung nach einem der Ansprüche 1 bis 6 oder pharmazeutische Zusammensetzung nach Anspruch 7 zur Verwendung als Arzneimittel.

9. Verbindung nach einem der Ansprüche 1 bis 6 oder pharmazeutische Zusammensetzung nach Anspruch 7 zur Verwendung bei der Vorbeugung oder Behandlung einer Erkrankung, die durch die Modulation von Neurotransmitterwerten gelindert wird, wobei die Erkrankung ausgewählt ist aus der Gruppe bestehend aus: Hypotonie, orthostatische Hypotonie, neurogene orthostatische Hypotonie, symptomatische neurogene orthostatische Hypotonie, neurogene orthostatische Hypotonie in Verbindung mit multipler Systematrophie (MSA), orthostatische Hypotonie in Verbindung mit dem Shy-Drager-Syndrom, neurogene orthostatische Hypotonie in Verbindung mit familiärer amyloider Polyneuropathie (FAP), neurogene orthostatische Hypotonie in Verbindung mit reinem autonomen Versagen (PAF), idiopathische orthostatische Hypotonie, asympathikotone Hypotonie, neurogene orthostatische Hypotonie in Verbindung mit Morbus Parkinson, intradialytische Hypotonie (IDH), hämodialysebedingte Hypotonie, Hypotonie in Verbindung mit Fibromyalgie-Syndrom (FMS), Hypotonie bei Rückenmarksverletzungen, Hypotonie in Verbindung mit chronischem Erschöpfungssyndrom (CFS), Gangstörungen (Freezing), Akinesie und Dysarthrie bei Morbus Parkinson, Lewy-Körperchen-Demenz, REM-Schlaf-Verhaltensstörungen, chronische Herzinsuffizienz, stressbedingte Erkrankungen, motorische oder sprachliche Störungen, chronische Schmerzen, Schlaganfall, zerebrale Ischämie, Nasenschleimhautschwellung, Stimmungsschwankungen, Schlafstörungen, Narkolepsie, Schlaflosigkeit, Aufmerksamkeitsdefizitstörung (ADD), Aufmerksamkeitsdefizit-Hyperaktivitätsstörung (ADHD), Anosmie, Hyposmie, leichte kognitive Beeinträchtigung (MCI), Down-Syndrom, Alzheimer-Krankheit, durch die Parkinson-Krankheit verursachte Haltungsreflexanomalien, autonome Autoimmunerkrankungen, familiäre Dysautonomie, diabetische autonome Neuropathie, Amyloidose bei multiplem Myelom, Morbus Parkinson, proprandiale Hypotonie, Dopamin-Beta-Hydroxylase-Mangel, Schmerzen, progressive supranukleäre Lähmung, Menkes-Syndrom, familiäre Dysautonomie (Riley-Day-Syndrom), PD-bedingte Dysautonomie (autonome Dysfunktion), orthostatische Intoleranz bei Jugendlichen, neurokardiogene Synkope (vasovagal), posturales orthostatisches Tachykardiesyndrom (POTS), Fibromyalgie, Allodynie, Hyperalgesie, Erschöpfung, Schlafstörung, Depression, chronische orthostatische Intoleranz, pädiatrische Entwicklungsstörungen, genetische Erkrankungen, die mit einer verminderten Noradrenalin-Synthese oder -Wirkungen einhergehen, Multisystem-Regulationsstörungen, Schmerzen, neurodegenerative Erkrankungen, kognitive Dysfunktion, Geruchsstörungen, neuroendokrine Erkrankungen und Autoimmunerkrankungen; wobei die Erkrankung vorzugsweise ausgewählt ist aus der Gruppe bestehend aus orthostatischer Hypotonie, neurogener orthostatischer Hypotonie in Verbindung mit multipler Systematrophie (MSA), orthostatischer Hypotonie in Verbindung mit Shy-Drager-Syndrom, neurogener orthostatischer Hypotonie in Verbindung mit familiärer amyloider Polyneuropathie (FAP), neurogener orthostatischer Hypotonie in Verbindung mit reinem autonomen Versagen (PAF), idiopathische orthostatische Hypotonie, asympathikotone Hypotonie, neurogene orthostatische Hypotonie in Verbindung mit Morbus Parkinson, intradialytische Hypotonie (IDH), Hämodialyse-induzierte Hypotonie, Hypotonie in Verbindung mit Fibromyalgie-Syndrom (FMS), Hypotonie bei Rückenmarksverletzungen und Hypotonie in Verbindung mit chronischem Erschöpfungssyndrom (CFS) und am meisten bevorzugt, wenn es sich bei der Erkrankung um orthostatische Hypotonie handelt.

10. Verbindung nach einem der Ansprüche 1 bis 6 oder pharmazeutische Zusammensetzung nach Anspruch 7 zur Verwendung bei der Vorbeugung oder Behandlung einer Erkrankung, die durch die Modulation von Neurotransmitterwerten gelindert wird, wobei die Erkrankung ausgewählt ist aus der Gruppe bestehend aus Dopamin-beta-Hydroxylase-Mangel, Menkes-Syndrom, Vitamin-C-Mangel, Lewy-Körperchen-Erkrankungen, Morbus Parkinson, Lewy-Körper-Demenz, reines autonomes Versagen, familiäre Dysautonomie, Zustand nach bilateraler endoskopischer thorakaler Sympathektomie, orthostatische Intoleranz und orthostatische Hypotonie.

11. Verbindung nach einem der Ansprüche 1 bis 6 oder pharmazeutische Zusammensetzung nach Anspruch 7 zur Verwendung gemäß Anspruch 9 oder Anspruch 10, wobei die Verbindung oder pharmazeutische Zusammensetzung in Kombination mit einem zusätzlichen therapeutischen Mittel verwendet wird.

12. Verbindung nach einem der Ansprüche 1 bis 6 oder pharmazeutische Zusammensetzung nach Anspruch 7 zur Verwendung gemäß Anspruch 11, wobei das zusätzliche therapeutische Mittel ausgewählt ist aus der Gruppe bestehend aus: 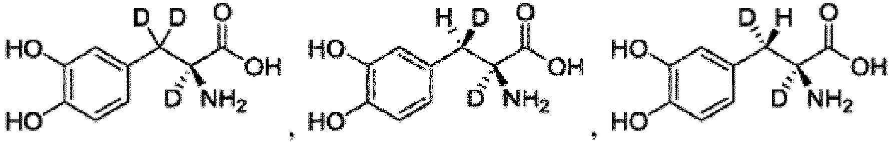 und Gemischen davon.

## Revendications

1. Composé enrichi en deutérium qui est :

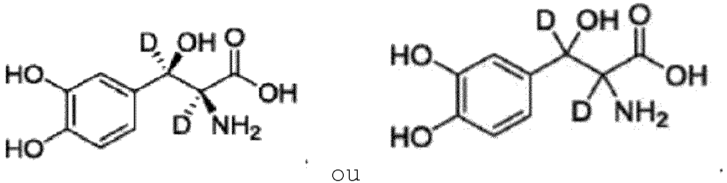

2. Composé selon la revendication 1, qui est :

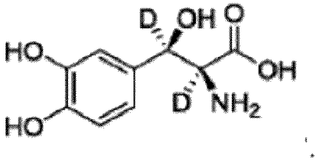

3. Composé selon la revendication 1 ou la revendication 2, chaque position représentée comme D possédant un enrichissement en deutérium de pas moins de 10 %.

4. Composé selon l'une quelconque des revendications précédentes, chaque position représentée comme D possédant un enrichissement en deutérium de pas moins de 50 %.

5. Composé selon l'une quelconque des revendications précédentes, chaque position représentée comme D possédant un enrichissement en deutérium de pas moins de 90 %.

6. Composé selon l'une quelconque des revendications précédentes, chaque position représentée comme D possédant un enrichissement en deutérium de pas moins de 98 %.

7. Composition pharmaceutique comprenant un support pharmaceutiquement acceptable conjointement avec un composé selon l'une quelconque des revendications précédentes.

8. Composé selon l'une quelconque des revendications 1 à 6, ou composition pharmaceutique selon la revendication 7, pour une utilisation en tant que médicament.

9. Composé selon l'une quelconque des revendications 1 à 6, ou composition pharmaceutique selon la revendication 7, pour une utilisation dans la prévention ou le traitement d'un trouble amélioré par la modulation des niveaux de neurotransmetteurs, ledit trouble étant choisi dans le groupe constitué par : hypotension, hypotension orthostatique, hypotension orthostatique neurogénique, hypotension orthostatique neurogénique symptomatique, hypotension orthostatique neurogénique associée à une atrophie multiple du système (MSA), hypotension orthostatique associée au syndrome de Shy-Drager, hypotension orthostatique neurogénique associée à une polyneuropathie amyloïde familiale (FAP), hypotension orthostatique neurogénique associée à une insuffisance autonome pure (PAF), hypotension orthostatique idiopathique, hypotension asympathicotonique, hypotension orthostatique neurogénique associée à la maladie de Parkinson, hypotension intradialytique (IDH), hypotension induite par l'hémodialyse, hypotension associée au syndrome de fibromyalgie (FMS), hypotension dans les lésions de la moelle épinière, hypotension associée au syndrome de fatigue chronique (CFS), démarche gelée, akinésie et dysarthrie dans la maladie de Parkinson, démence à corps de Lewy, trouble du comportement à mouvement oculaire rapide (REM), insuffisance cardiaque chronique, troubles liés au stress, troubles moteurs ou de la parole, douleur chronique, accident vasculaire cérébral, ischémie cérébrale, congestion nasale, troubles de l'humeur, troubles du sommeil, narcolepsie, insomnie, trouble déficitaire de l'attention (ADD), trouble déficitaire de l'attention avec hyperactivité (ADHD), anosmie, hyposmie, déficience cognitive légère (MCI), syndrome de Down, maladie d'Alzheimer, anomalie du réflexe postural causée par la maladie de Parkinson, insuffisance autonome auto-immune, dysautonomie familiale, neuropathie autonomique diabétique, amyloïdose dans le cadre de myélome multiple, maladie de Parkinson, hypotension préprandiale, déficience en bêta-hydroxylase dopamine, douleur, paralysie supranucléaire progressive, maladie de Menkes, dysautonomie familiale (syndrome de Riley-Day), dysautonomie liée à la PD (dysfonction autonome), intolérance orthostatique chez les adolescents, syncope neurocardiogène (vasovagale), syndrome de tachycardie orthostatique posturale (POTS), fibromyalgie, allodynie, hyperalgésie, fatigue, troubles du sommeil, dépression, intolérance orthostatique chronique, troubles du développement pédiatrique, maladies génétiques entraînant une diminution de la synthèse ou des effets de la norépinéphrine, troubles multisystémiques de la régulation, douleur, maladies neurodégénératives, dysfonctionnement cognitif, troubles olfactifs, troubles neuroendocriniens et troubles autoimmuns ; préférablement, ledit trouble étant choisi dans le groupe constitué par hypotension orthostatique, hypotension orthostatique neurogénique associée à une atrophie multiple du système (MSA), hypotension orthostatique associée au syndrome de Shy-Drager, hypotension orthostatique neurogénique associée à une polyneuropathie amyloïde familiale (FAP), hypotension orthostatique neurogénique associée à une insuffisance autonome pure (PAF), hypotension orthostatique idiopathique, hypotension asympathicotonique, hypotension orthostatique neurogénique associée à la maladie de Parkinson, hypotension intradialytique (IDH), hypotension induite par l'hémodialyse, hypotension associée au syndrome de fibromyalgie (FMS), hypotension dans les lésions de la moelle épinière et hypotension associée au syndrome de fatigue chronique (CFS) ; et de préférence ledit trouble étant une hypotension orthostatique.

10. Composé selon l'une quelconque des revendications 1 à 6, ou composition pharmaceutique selon la revendication 7, pour une utilisation dans la prévention ou le traitement d'un trouble amélioré par la modulation des niveaux de neurotransmetteurs, ledit trouble étant choisi dans le groupe constitué par carence en dopamine-bêta hydroxylase, maladie de Menkes, manque de vitamine C, maladies à corps de Lewy, maladie de Parkinson, démence à corps de Lewy, insuffisance autonome pure, dysautonomie familiale, sympathectomie thoracique endoscopique bilatérale status-post, intolérance orthostatique et hypotension orthostatique.

11. Composé selon l'une quelconque des revendications 1 à 6, ou composition pharmaceutique selon la revendication 7, pour une utilisation selon la revendication 9 ou la revendication 10, le composé ou la composition pharmaceutique étant utilisé(e) en combinaison avec un agent thérapeutique supplémentaire.

12. Composé selon l'une quelconque des revendications 1 à 6, ou composition pharmaceutique selon la revendication 7, pour une utilisation selon la revendication 11, l'agent thérapeutique supplémentaire étant choisi dans le groupe constitué par 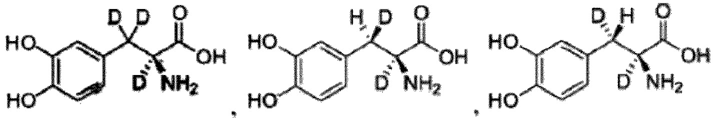 et des mélanges correspondants.
